(19) Europäisches Patentamt
European Patent Office
Office européen des brevets

(11) EP 1 246 640 B1

(12) **EUROPEAN PATENT SPECIFICATION**

(45) Date of publication and mention of the grant of the patent:
**19.04.2006 Bulletin 2006/16**

(21) Application number: **00986923.1**

(22) Date of filing: **19.12.2000**

(51) Int Cl.:
***A61K 38/40*** (2006.01) ***A61K 38/01*** (2006.01)
***A61L 2/16*** (2006.01) ***A01N 63/02*** (2006.01)
***A61K 45/00*** (2006.01) ***A61P 31/04*** (2006.01)

(86) International application number:
**PCT/CA2000/001517**

(87) International publication number:
**WO 2001/045732 (28.06.2001 Gazette 2001/26)**

(54) **METHOD AND COMPOSITION FOR TREATMENT AND/OR PREVENTION OF ANTIBIOTIC-RESISTANT MICROORGANISM INFECTIONS**

METHODE UND ZUSAMMENSETZUNG ZUR BEHANDLUNG UND/ODER PROPHYLAXE VON ANTIBIOTIKUM-RESISTENTEN-MIKROORGANISMUS INFEKTIONEN

PROCEDE ET COMPOSITION POUR LE TRAITEMENT ET/OU LA PREVENTION D'INFECTIONS AUX MICRO-ORGANISMES RESISTANTS AUX ANTIBIOTIQUES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **20.12.1999 US 172577 P**

(43) Date of publication of application:
**09.10.2002 Bulletin 2002/41**

(73) Proprietor: **Sa Majesté la Reine du Chef du Canada Agriculture;**
**et Agroalimentaire Canada**
**2000 Route 108 Est Lennoxville,**
**Québec J1M 1Z3 (CA)**

(72) Inventors:
- **DIARRA, Moussa, S.**
  **Fleurimont, Québec J1E 3P8 (CA)**
- **LACASSE, Pierre**
  **Lennoxville, Québec J1M 1L4 (CA)**
- **PETITCLERC, Denis**
  **Lennoxville, Québec J1M 2G4 (CA)**

(74) Representative: **Hinterberg, Katherine et al**
**Cabinet Germain et Maureau,**
**BP 6153**
**69466 Lyon Cedex 06 (FR)**

(56) References cited:
**EP-A- 0 629 347** **WO-A-98/40401**
**US-A- 5 198 419**

- **DATABASE WPI Section Ch, Week 199517 Derwent Publications Ltd., London, GB; Class B04, AN 1995-126103 XP002171004 & JP 07 048274 A (IMMUNO JAPAN KK), 21 February 1995 (1995-02-21)**
- **KUIPERS M E ET AL: "Synergistic fungistatic effects of lactoferrin in combination with antifungal drugs against clinical Candida isolates." ANTIMICROBIAL AGENTS AND CHEMOTHERAPY, (1999 NOV) 43 (11) 2635-41., XP001010102**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 246 640 B1

## Description

## BACKGROUND OF THE INVENTION

(a) Field of the Invention

The present invention relates to composition and uses for treating antibiotic-resistant microbial infections by administration of bovine lactoferrin or its metabolized form, the lactoferricin, alone or in combination with antibiotics or other families of antimicrobial products.

(b) Description of Prior Art

### Antibiotic Use in Animal Husbandry and Resistance

Two important factors impact on the emergence and spread of antibiotic resistance: transferable resistance genes and selective pressure by use of antibiotics. Besides hospitals with a concentration of patients prone to infections and corresponding antibiotic use, animal husbandry is a second considerable reservoir of heavy antibiotic use and transferable antibiotic resistance. Industrial animal husbandry keeps large numbers of animals in comparably small space .and outbreaks of infections can easily spread. For technical reasons there is often mass medication of all the animals of a particular flock or herd animals are also under transport stress when shipped from breeding stations to farms for fattening. The consequence is a broad scale antibiotic prophylaxis.

For a number of decades, antimicrobials have been used as growth promoters, especially in pig and poultry farming. The use of growth promoters leads to 4 - 5 % more body weight for animals receiving them as compared to controls. Much larger amounts of antibiotics are used in this manner than are used in medical applications: In Denmark in 1994, 24 kg of the glycopeptide vancomycin were used for human therapy, whereas 24,000 kg of a similar glycopeptide avoparcin were used in animal feed. From 1992 to 1996, Australia imported an average of 582 kg of vancomycin per year for medical purposes and 62,642 kg of avoparcin per year for animal husbandry. Vancomycin and avoparcin have the same mode of action; resistance to one can confer resistance to the other. The biological bases of the growth promoting effects are far from being understood; according to data from Sweden, this effect can be mainly demonstrated under sub-optimal conditions of animal performance.

That antibiotic use in agriculture will result in transfer of antibiotic resistant microorganism and transferable resistance genes to humans was already discussed nearly 30 years ago, especially with regard to growth promoters. At this time, it has been mentioned that there should be no use of antibiotics as growth promoters if they are also used for human chemotherapy and/or if they select for cross-resistance against antibiotics used in humans.

During the past 10 years, methods of molecular fingerprinting microbial pathogens and their resistance genes became a powerful tool for epidemiological tracing and have provided much more conclusive evidence for the spread of antibiotic resistance from animal husbandry to humans. Currently two issues are subjects of discussions among the scientific community and agriculture industry: antimicrobial growth promoters and veterinary use of fluoroquinolone.

That the comparably low concentrations of growth promoters select for transferable antibiotic resistance has often been doubted. There is however convincing evidence from two sets of studies. Feeding of oxytetracycline to chickens was shown to select for plasmid mediated tetracycline resistance in *E. coli* in chickens. Transfer of the tetracycline resistant *E. coli* from chickens to farm personnel was demonstrated. In some countries, oxytetracycline was replaced as feed additive by the streptothricin antibiotic nourseothricin. This antibiotic was used country wide only for animal feeding.

In 1985, resistance (mediated by a transposon-encoded streptothricin acetyltransferase gene) was found in *E. coli* from the gut of pigs and in meat products. By 1990, resistance to nourseothricin had spread to *E. coli* from the gut flora of pig farmers, their families, citizens from municipal communities, and patients with urinary tract infections. In 1987, the same resistance determinant was detected in other enteric pathogens, including *Shigella* that occurs only in humans.

With the emergence and spread of glycopeptide resistance, Enterococci became a subject of great interest. Enterococci colonize the guts of humans and other animals, and easily acquire antibiotic resistance genes and transfer them. During the last 5 years, enterococci have been recorded among the top five of microbial nosocomial pathogens. Although less pathogenic than *E. faecalis, E. faecium* has drawn increased attention because of its development of resistance to glycopeptides. In enterococci there are three known genotypes of transferable glycopeptide resistance with the vanA gene cluster the most widely disseminated one. Studies demonstrating selection of transferable, vanA-mediated glycopeptide resistance in *E. faecium* by use of the glycopeptide avoparcin as a growth promoter in animal husbandry have again focused attention on the use of antimicrobials as growth promoters. Glycopeptide resistant *E. faecium* (GREF) can easily reach humans via meat products and consequently GREF have been isolated from stool specimens from nonhospitalized humans. A common structure of the vanA gene cluster has been found in a number of GREF of different ecological origin (human, food, and animals), indicating a frequent dissemination of vanA among different strains and also among different conjugative plasmids.

Ergotropic use of avoparcin was stopped in European countries between 1995 and 1997. When investigated for GREF by end of 1994, thawing liquid from all of the investigated poultry carcasses was found heavily contaminated. By end of 1997, GREF were found in comparably low number in only 25 % of the investigated samples. In parallel a decrease of fecal carriage of GREF by humans in the community was seen: 12 % by end of 1994 and 3.3 % by end of 1997. These findings highlight the potential role of a reservoir of transferable glycopeptide resistance in animal husbandry for spread to humans. With the availability of the streptogramin combination quinupristin/dalfopristin streptogramins became an important alternative for treatment of infections with GREF (not *E. faecalis)*

Until last year, there was no medical use of streptogramins in German hospitals. However streptogramine resistance has been found in GREF from both patients and animals. The resistance is mediated by the satA gene coding for a streptogramin acetyltransferase. The dissemination of satA was probably driven by use of the streptogramin antibiotic virginiamycin as growth promoter for more than 20 years.

Veterinary fluoroquinolone use a decrease in fluoroquinolone sensitivity in *Salmonella typhimurium* has been described which parallels the time of fluoroquinolone use in veterinary medicine. This was especially observed in the United Kingdom for *S. typhimurium* strain DT 104. Although the MIC's of ciprofloxacin for these isolates (0.25 - 1.0 mg/ 1) are still below clinical breakpoints for fluoroquinolones for ciprofloxacin resistance (4 mg/1), the clinical failure of ciprofloxacin for treating infections with *S. typhimurium* exhibiting elevated MIC's raises concern with regard to enteric *Salmonella spp.*

Fluoroquinolone resistance in microorganism is mainly due to mutations in the target enzymes (DNA gyrase, topoisomerase IV) and therefore spreads in a clonal way with particular microbial strains affected. Enterics develop quinolone resistance by stepwise acquisition of mutations at certain positions in the active center of the target enzymes. Further accumulation of these mutations by enteric *Salmonella spp.* will very probably lead to high-level quinolone resistance.

Another intestinal pathogen that has its reservoir in animals is *Campylobacter* spp. Fluroquinolone resistant *Campylobacter* can be isolated from human infections; from fecal samples of chickens and from chicken meat. Different frequencies of quinolone resistant *Campylobacter* isolates from human cases of diarrhea have been reported from several parts of the world. The *Campylobacter spp.* are obviously polyclonal (several strains harbored in the gut flora of man and animals), comparable to *E. coli.* Although currently available molecular typing techniques are available to *Campylobacter* most probably because of polyclonality quinolone resistant *Campylobacter* strains have not been traced back to animal flocks.

Global situation for prevention and regulation use and licensing of these compounds varies tremendously worldwide. In developing countries, which are responsible for about 25 % of world-meat production, policies regulating veterinary use of antibiotics are poorly developed or absent. In China, raw mycelia are used as animal growth promoters. The problems caused by inappropriate use of antibiotic reach beyond the country of origin. Meat products are traded worldwide, and microbial populations evolve independent of geographical boundaries. Use of antimicrobials as growth promoters include an uncalculable hazard. As evident from the emergence of streptogramin resistance in enterococci, a compound or class of compounds that is used now as a growth promoter can, in the future, become important for human chemotherapy.

**Mechanisms of Antibiotic Resistance in Oral Microorganism**

The upper respiratory tract, including the nose, oral cavity, nasopharynx, and pharynx harbors a wide range of Gram-positive, Gram-negative cell-wall-free aerobic and anaerobic microorganism.

Oral microflora populations are not static. They change in response to the age, hormonal status, diet, and overall health of an individual. In addition, new and different microbes are ingested or inhaled daily. The exact composition of species will vary among individuals and, over time, in the same individual. An estimated 300 or more different species may be cultured from periodontal pockets alone, and up to 100 species may be recovered from a single site.

Such microbial microcosms provide an excellent opportunity for the transfer of antibiotic resistance genes. The normal microbial flora of the human body acts as a reservoir for such resistance traits. Gene exchange has been demonstrated among oral and urogenital species of microorganism, and between divergent oral microorganism under laboratory conditions. Prophylactic use of antibiotics before many dental procedures and for periodontal disease or oral abscess-infections that have not been shown to require antibiotic therapy contribute to the resistance reservoir. The β-lactams, tetracyclines, and metronidazole are the most commonly recommended and prescribed antibiotics. Macrolides, clindamycin, and fluoroquinolones are rarely used, while aminoglycosides are normally not recommended.

**Resistance to beta-lactam antibiotics**

Enzymatic resistance to the beta-lactam antibiotics is most often due to an enzyme, beta-lactamase, which hydrolyses amides, amidines, and other carbon and nitrogen bonds, inactivating the antibiotic. More than 190 unique beta-lactamases have been identified in Gram-positive and Gram-negative microorganism from the oral tract.

The first beta-lactamase in common oral microorganism was described on a plasmid in *Haemophilus influenzae* in the early 1970's. It carried the TEM-1 beta-lactamase first described in *E. coli.* The TEM-1 enzyme has been found *in H. parainfluenzae* and *H. paraphrohaemolyticus* and may be found in commensal *Haemophilus* species. The TEM-1 beta-lactamase is usually associated with large conjugative plasmids that are specific for the genus *Haemophilus,* which can also carry other genes for resistance to chloramphenicol, aminoglycosides and tetracycline.

At about the same time, *Neisseria gonorrhoeae* acquired TEM-1 beta-lactamase on small plasmids that can be mobilized to other strains by transfer plasmids in the strains. They are closely related to a susceptible *H. parainfluenzae* plasmid and small TEM beta-lactamase plasmids from *H. ducreyi* and *H. parainfluenzae.* Some have hypothesized that *H. parainfluenzae* may be the most likely ancestral source for these related TEM beta-lactamase plasmids. Plasmids from this group have been reported periodically in *Neisseria meningitidis* although no natural isolates have survived for independent testing. However, the small *N. gonorrhoeae* beta-lactamase plasmids can be transferred and maintained in *N. meningitidis* by conjugation under laboratory conditions.

TEM beta-lactamase has also been reported in a variety of commensal Neisseria species, usually found on small plasmids genetically related to the *E. coli* RSF1010 plasmid rather than the gonococcal plasmid. Similar plasmids have been found in *Eikenella corrodens.* These RSF1010-like plasmids may carry genes conferring resistance to sulfonamide or streptomycin singly or in combination. Larger plasmids from multi-resistant *N. sicca* have also been described, coding for resistance to tetracycline, a variety of aminoglycosides, and to the TEM beta-lactamases. Isolates of multi-resistant *Moraxella (Branhamella) catarrhalis,* initially reported to CDC for confirmation, were later identified as commensal Neisseria species.

A second beta-lactamase ROB has been described in *H. influenzae* on a small plasmid that is virtually identical to ROB-bearing plasmids found in a number of strictly animal microbial pathogens, including *Actinobacillus* and *Pasteurella* species.

More recently, strict anaerobic Gram-negative microorganism, including *Bacteroides forsythus, Fusobacterium nucleatum, Prevotella species, Porphyromonas asaccharolytica,* and *Veillonella* species, have been shown to carry genes for β-lactamases. Only some of the enzymes have been characterized, and the genetic location (plasmid vs. chromosome) has generally not been determined.

Non-enzymatic resistance to penicillin in naturally transformable microorganism *(Haemophilus, Neisseria, Streptococcus)* can be due to replacement of parts of the genes encoding for penicillin-binding proteins (PBP), the targets of penicillin, with corresponding regions from more resistant species. This mechanism of resistance is less common than is resistance caused by beta-lactamases. For *N. meningitidis,* these more resistant regions of the PBP genes are closely related to the genes of commensal *N. flavescens* and *N. cinera.* One of the PBP genes, penA, has been shown to be very diverse, with 30 different mosaic genes found among 78 different isolates examined. The mosaics PBPs in *S. pneumoniae* have regions from *S. mitis* as well as from unknown streptococcal species.

Another non-enzymatic resistance mechanism, found in methicillin-resistant *S. aureus,* is the mecA gene, a genetic determinant which codes for an additional low-affinity penicillin-binding protein, PBP2a, and lies on a 30 to 40 kb DNA element that confers an intrinsic resistance to beta-lactams. Among 15 different species of *Staphylococcus* screened for the mecA gene, 150 isolates of *Staph. sciuri* hybridized to the gene. Because not all *Staph. sciuri* are penicillin resistant, the *Staph. sciuri* mecA homologue may perform a normal physiological function in its natural host unrelated to beta-lactam resistance.

**Tetracycline resistance**

Eighteen distinguishable determinants for tetracycline resistance have been described that specify primarily two mechanisms of resistance: efflux and protection of ribosomes. The distribution of the different Tet determinants varies widely, related in part to the ease of transfer of particular Tet determinants between various isolates and genera. The Tet B gene has the widest host range among the Gram-negative efflux genes and has been identified in a number of oral species. Both *Actinomyces actinomycetemcomitans* and *Treponema denticola* have been associated with periodontal disease. The TetB determinant is found on conjugative plasmids in *Actinobacillus* and *Haemophilus* species. The plasmid carrying tet (B) from *A. actinomycetemcomitans* was transferable to *H. influenzae.* The TetB determinant was not mobile in the small number of *Moraxella* and *Treponema* isolates examined.

Recently, the Gram-positive efflux-mediated genes [tet (K) and tet (L)] in a few oral Gram-negative microorganism was found. *Haemophilus aphrophilus,* isolated from periodontal patients in the 1990s, carried the tet (K) gene. A few isolates of *V. parvula* have been found that carry tet (L) or tet (Q); however, most of the isolates examined carry the tet (M) gene. Oral streptococci may carry multiple different tet genes, and tet (M), tet (Q), tet (K), and tet (L) have all been found in streptococci, singly or in combination. Recently, other ribosomal protection genes [tet (U), tet (S) and tet (T)] have been found in enterococci. Tet (S) has been found in *Streptococcus milleri* and tetracycline-resistant streptococci have been isolated that do not carry any of the known tet genes. Tet (M), which produces a ribosome-associated protein, is widely distributed in both Gram-positive and Gram-negative genera.

The tet (Q) ribosomal protection gene was first found in colonic Bacteroides and has usually been found in Gram-negative anaerobic species that are related to *Bacteroides,* such as *Prevotella.* A few isolates of V. *parvula* have been found to carry tet (Q); however, most of the isolates characterized carry tet (M). Oral *Mitsuokella* and *Capnocytophaga* also carry tet (Q).

**Other resistance mechanisms**

Metronidazole resistance has been reported in oral microorganism, but the genetic basis is not known. In colonic Bacteroides, four genes, nimA, nimB, nimC and nimD, have been described and sequenced. They are located on either the chromosome or a variety of plasmids, confering a range of resistance. The nim genes likely code for a 5-nitroimidazole reductase that enzymatically reduces 5-nitroimidazole to a 5-amino derivative.

Enzymes that acetylate, phosphorylate, or adenylate aminoglycosides have been characterized in *S. pneumoniae,* other streptococci, staphylococci, and, more recently, commensal *Neisseria* and *Haemophilus* species. An isolate of *Campylobacter ochraceus* has been found that is resistant to aminoglycosides, chloramphenicol, and tetracycline.

Early isolates of erythromycin-resistant *S. pneumoniae* carried the Erm B class of rRNA methylases, which modifies a single adenine residue in the 23S RNA conferring resistance to macrolides, lincosamides and streptogramin B. rRNA methylases have been identified in *A. actinomycetemcomitans* and *Campylobacter rectus.* In both species, the rRNA methylases are associated with conjugative elements that can be transferred to *Enterococcus faecalis* and from *A. actinomycetemcomitans* to *H. influenzae.* Many other oral microorganism have been reported to be resistant to erythromycin or clindamycin.

Microorganism making up the oral flora are reservoirs of important antibiotic resistance traits. Their emergence reflects the overuse and misuse of antibiotics and their potential for transfer of these traits to other more pathogenic species.

**Antibiotic Use in Plant Disease Control**

A wide range of food crops and ornamental plants are susceptible to diseases caused by microorganism. In the 1950s, soon after the introduction of antibiotics into human medicine, the potential for these "miracle drugs" to control plant diseases was recognized. Unfortunately, just as the emergence of antibiotic resistance sullied the miracle in clinical settings, resistance has, also limited the value of antibiotics in crop protection. In recent years, antibiotic use on plants, and its potential impact on human health, an emergence of resistances have been observed in several countries.

**Streptomycin resistance occurs in plant pathogens.**

Studies have not revealed oxytetracycline resistance in plant pathogenic microorganism but have identified tetracycline-resistance determinants in nonpathogenic orchard microorganism. Two genetically distinct types of streptomycin resistance have been described: a point mutation in the chromosomal gene rpsL which prevents streptomycin from binding to its ribosomal target (MIC >1,000 mg/ml); or inactivation of streptomycin by phosphotransferase, an enzyme encoded by strA and strB (MIC 500-750 mg/ml). The genes strA and strB usually reside on mobile genetic elements and have been identified in at least 17 environmental and clinical microorganism populating diverse niches.

Because antibiotics are among the most expensive pesticides used by fruit and vegetable growers, and their biological efficacy is limited, many growers use weather-based disease prediction systems to ensure that antibiotics are applied only when they are likely to be most effective. Growers can also limit antibiotic use by planting disease resistant varieties and, in some cases, using biological control (applying saprophytic microorganism that are antagonistic to pathogenic microorganism). Despite these efforts to reduce grower's dependency on antibiotics, these chemicals remain an integral part of disease management, especially for apple, pear, nectarine and peach production.

**Special Aspects of Plant Antibiotic Use**

Although antibiotic use on plants is minor relative to total use, application of antibiotics in the agroecosystem presents unique circumstances that could impact the build-up and persistence of resistance genes in the environment.

In regions of dense apple, pear, nectarine or peach production, antibiotics are applied to hundreds of hectares of nearly contiguous orchards. The past decade has seen a dramatic increase in the planting of apple varieties and rootstocks that are susceptible to the devastating microbial disease, fire blight. This has created a situation analogous to clinical settings where immune-compromised patients are housed in crowded conditions--settings associated with the proliferation and spread of antibiotic-resistance genes.

Second, the purity of antibiotics used in crop protection is unknown. Reagent and veterinary grade antibiotics have been found to contain antibiotic resistance genes from the producing *Streptomyces* spp. Plant-grade antibiotics are unlikely to be purer than those used for treating animals and may themselves be an origin of antibiotic resistance genes in agroecosystems. The genes that were amplified from antibiotics, otrA and aphE, are different from the resistance genes strA and strB that have been described in plant-associated microorganism. Thus, it may be that plant-grade antibiotics are a potential origin of resistance genes in the environment, but are not necessarily present and active in plant pathogenic microorganism.

The evolution of antibiotic resistant microorganism is outpacing the discovery of new antimicrobial products.

**The Role of Selective Antibiotic Concentrations on the Evolution of Antimicrobial Resistance**

A single gene encoding the widespread TEM-1 (or TEM-2) beta-lactamase, hydrolyzing ampicillin, was changed in different ways so that now the enzyme is now able to inactivate third generation cephalosporins or monobactams. Modifications in a gene encoding a penicillin-binding protein (PBP2) in *Streptococcus pneumoniae* has provoked the frightening threat of beta-lactam resistance in the most common microbial pathogen in the respiratory tract. When the "new" TEM or PBP genes involved in resistance were sequenced, it was frequently found that several mutations were present in the gene, suggesting that a cryptic evolution had occurred. That implies that each one of the 'previous' mutational events was in fact selected, and the resulting enrichment of the harboring microbial clone favored the appearance of new, selectable mutations. In most cases, conventional antibiotic susceptibility tests failed to detect early mutations increasing only in a very modest amount the minimal inhibitory concentration (MIC) of the organism. In such a way, the use of the selecting antibiotic was non-discontinued and the mutation was selected.

Not only clinicians, but also microbiologists have frequently disregarded the importance of "low-level resistance," as it was assumed that the mutants exhibiting low MICs were unselectable, considering the high antibiotic concentrations attainable during treatments.

At any dosage, antibiotics create concentration gradients, resulting from pharmacokinetic factors such as the elimination rate of the tissue distribution. Most probably, the microbial populations are facing a wide range of antibiotic concentrations after each administration of the drug. On the other hand, the spontaneous variability of microbial populations may provide a wide possibility of potentially selectable resistant variants. Which is the antibiotic concentration able to select one of these resistant variants?

The answer is simple: any antibiotic concentration is potentially selective of a resistant variant if it is able to inhibit the susceptible population but not the variant harboring a mechanism of resistance. In other words, a selective antibiotic concentration (SAC) is such if exceed the minimal inhibitory concentration (MIC) of the susceptible population, but not that corresponding (even if it is very close) to the variant population. If the MICs of both the susceptible and the variant populations is surpassed, no selection takes place; and the same is true if the antibiotic concentration is below the MICs of both populations. Therefore, the selection of a particular variant may occur in a very narrow range of concentrations.

The continuous variation of antibiotic concentrations may resemble a tuning device which 'selects' at a particular radio frequency a particular emission. Under or over such a frequency the emission is lost. The 'valley' between the MICs of the susceptible and the resistant variant populations is the 'frequency signal' recognized by the SAC.

Because of the natural competition of microbial populations in a closed habitat, the 'signal' is immediately amplified. The fit mutant shows an intensive, distinctive reproduction rate at the expense of the more susceptible population, leading to a quantum modification of the culture, as could be predicted by the periodic selection.

The above-proposed way on the effect of SACs was tested in laboratory using mixed cultures of susceptible and resistant microbial populations. A dense culture of an *Escherichia coli* strain harboring a wild TEM-1 beta-lactamase was mixed with their homogenic derivatives (obtained by directed mutagenesis) harboring the beta-lactamases TEM-12 (a single amino acid replacement with respect to the TEM-1 enzyme) and TEM-10 (a single amino acid replacement with respect to TEM-12). The relative proportions of the three strains were 90:9:1 of the total population. The mixture was incubated during 4 h with different antibiotic concentrations, and the composition of the total population was then analyzed by subculture. At a very low cefotaxime concentration, 0.008 μg/ml, TEM-12 (conventional MIC=0.06 μg/ml) began to be selected against TEM-1 (MIC=0.03 μg/ml), reached a maximal selection (nearly 80% of total population) at 0.03 μg/ml and was again displaced by TEM-1 at 0.06 μg/ml. At this turn, TEM-1 was displaced by TEM-10 (MIC=0.25 μg/ml) at 0.12 μg/ml. As predicted, TEM-12 was only selected within a narrow concentration range. Therefore, low antibiotic concentrations efficiently select low-level resistant mutants. As far as this population is enriched, it may serve as a new source of secondary genetic variants (for instance TEM-10 can give rise to TEM-12), which were subsequently selected against the predominant population in new (higher) concentration intervals. Similar results were obtained when mixed susceptible and resistant *Streptococcus pneumoniae* populations were challenged with different low beta-lactam concentrations: intermediate resistant strains were selected over the predominant susceptible population only at discrete low-level concentrations.

Selection with high antibiotic concentrations will only give rise to high-level resistant variants. But during treatments, low-level concentrations, particularly in the so-called long-acting drugs, occurs with a higher frequency than the high-level ones, both in terms of time (duration) and space (different colonized locations in the human body), and therefore its overall selective power is certainly higher. Any treatment produces a low-level potentially selective antibiotic concentration for resistant microorganism.

Microbiology has been more concerned about the mechanisms of resistance than for population genetics or the evolutionary processes leading to the appearance and spread of antimicrobial resistance. The time is arrived to propose evolutionary products, serving the scientifically support measures against the environmental health damage produced by antibiotics.

The resistant population (R) will be efficiently selected over the susceptible one (S) in a short range of selective concentrations (in this case, 0.1-0.2 μg/ml). Concentrations below or over this range are non-selective. A low concentration (0.01 μg/ml) will "select" both S and R populations; a higher concentration (2 μg/ml) will "counterselect" both S and R. In both cases, the selective power for R populations is very low. Selection takes place at particular concentrations (selective antibiotic concentrations or SACs).

## Treatment of mastitis

*Staphylococcus aureus,* is an important human and animal pathogen that causes superficial, deep-skin, soft-tissue infection, endocarditis, and bacteremia with metastatic abscess formation and a variety of toxin-mediated diseases including gastroenteritis, scalded-skin syndrome and toxic shock syndrome. This microorganism is also the most common cause of bovine mastitis which is a disease that causes important losses in milk production. Coliforms *(Escherichia coli, Klebsiella* spp. *Enterobacter* spp *citrobacter* spp), *streptococci (S. agalactiae, S. dysgalactia, S. uberis, enterococci)* and *Pseudomonas* spp are also isolated from bovine mastitis. It is generally agreed that these pathogens are capable of producing many factors of virulence. *S. aureus* is able to produce a range of toxins and hemolysins. During mammary gland infection, *S. aureus* adhere to the glandular epithelium that is followed by erosion, local invasion, and a diffuse exudative inflammatory reaction accompanied by systemic symptoms.

Despite the progress in antimicrobial therapy, the treatment and prevention of staphylococcal infection remains a clinical problem. β-lactams antibiotics are the best weapons against staphylococci. However, the widespread use of β-lactam antibiotics has lead to a dramatic increase of β-lactamase producing strains of *S. aureus.* For example, this bacterium is able to produce four types (A, B, C and D) of β-lactam hydrolytic enzymes (β-lactamase) which allow it to be resistant to β-lactam antibiotics. Currently, 80 to 90% of *S. aureus* isolated in hospital and about 75 % isolated from bovine intramammary infections produce β-lactamase. This enzyme contributes to the pathogenesis of *S. aureus* infection and reduces the efficacy of antibiotic prophylaxis. In staphylococcal mastitis, bovine demonstrates poor clinical and bacteriologic response to standard antibiotic. When acute infection seems to respond to antibiotherapy, chronic relapsing disease characterized by long periods of quiescence between episodes of acute illness may occur. This phenomenon makes control of *S. aureus* infections difficult and there is limited information on the possible host defense against this pathogen during infection.

Products and methods of the present invention involve substantially non-toxic compounds available in large quantities by means of synthetic or recombinant methods. LF and LFC have microbicidal or bacteriostatic activity when administered or applied as the sole antimicrobial agent. Such compounds ideally are useful in combinative therapies with other antimicrobial agents, particularly where potentiating effects are provided.

Lactoferrin (LF) is a 80-kDa and lactoferricin (LFC) a pepsin hydrolysat of LF. Lactoferrin is an iron-binding glycoprotein found in milk of many species including human and cow. It is also present in exocrine fluids such as bile, saliva and tear. Both mammary epithelial and polymorphonulear cells can release this protein. Migration of leukocytes into milk during infection is accompanied by a spectacular increase of LF concentration in milk. The presence of LF in specific granules of neutrophils and its release in inflammatory reaction has been considered to play a role in immunomodulation. LF has also been shown to bound DNA, which can lead to the transcriptional activation of diverse molecules. Many reports identify LF as an important factor in host defense against infection and excessive inflammation. This protein in its iron-limited form, has been shown to inhibit the growth of many pathogenic microorganism. It was demonstrated the ability of LF to promote growth of *Bifidobacterium* spp independently to its iron level. The binding of iron in the media is the most well-know mechanism by which LF induces growth inhibition of microorganism. LF-mediated bacteriostasis of Gram-negative microorganism may also involve its interaction with lipid A of lipopolysaccharide (LPS), and with pore forming proteins (porins) of the outer membrane altering integrity and permeability of microbial wall. It has been suggested that the binding of LF to the anionic lipoteichoic acid of *Staphylococcus epidermidis* decreased the negative charge allowing greater accessibility of lysozyme to the peptidoglycan. Other antimicrobial mechanisms of LF or LFC have not been described in Gram positive bovine mastitis pathogens.

WO98/40401 claims compositions for treating infections and overcoming resistance using cationic peptides such as lactoferricin B, alone or in combination with antibiotics. EP 629347 discloses the use of lactoferrin hydrolysate and at least an antibiotic for treatment against bacteria, yeast, and fungi.

The relationship between microorganism, host and antibiotic can be very complex. An antibiotic should combine good antimicrobial activity and the capacity to work in association with the host defense systems. Nevertheless, the *in vitro* determination of susceptibility of microorganism to an antibiotic does not account for its interactions with the host defenses and its pharmacodynamic parameters such as post antibiotic effect on microorganism. The purpose of the present work was to investigate the physiological effects of bovine apo-lactoferrin or its pepsine hydrolysat (lactoferricin) alone or in combination with traditional antibiotics on both Gram positives (*S. aureus*) and Gram negatives (*E. coli* and *K. pneumioniae*) microbial strains isolate from bovine mammary gland. Results indicate that lactoferrin can affect to staphylococcal cells, and increase the inhibitory activity of usual antibiotic at varying degrees.

It would be highly desirable to be provided with a means to reverse the resistance to antibiotic of antibiotic-resistant microorganisms in the treatment and/or prevention of infections caused by these microrganisms.

## SUMMARY OF THE INVENTION

One aim of the present invention is to provide means to counteract the development of and to reverse the resistance to antibiotic of antibiotic-resistant microorganism in the treatment and/or prevention of infection caused by these microorganisms.

One aim of the present invention is to provide efficient drug formulations in order to treat and prevent infectious diseases caused by pathogenic antibiotic-resistant microorganism in animals, including human being. Another object is to provide a new use to treat and prevent microbial diseases and to potentiate the efficacy of antibiotics, including conditions associated therewith or resulting therefrom, in a subject by using the LF or LFC alone, or in combination with an antibiotic. The invention is based upon the discovery that LF and LFC have direct microbicidal and growth inhibitory effects on some antibiotic-resistant microorganisms, and that LF and LFC unexpectedly have the ability to reverse the antibiotic resistance of antibiotic-resistant microorganism. The invention is also based upon the finding that LF and LFC in combination with antibiotics provide additive and synergistic microbicidal/growth inhibitory effects when used concurrently.

According to one aspect of the invention, a use is provided of treating an antibiotic-resistant microbial infection comprising the step of administration to a subject suffering from an antibiotic-resistant microbial infection of the LF or LFC in an amount sufficient for therapeutic effectiveness. This may be practiced when any LF or LFC susceptible antibiotic-resistant microbial species is involved in the infection.

A second aspect of the invention provides a treatment of antibiotic-resistant microbial infection by concurrently administration to a subject suffering from an antibiotic-resistant microbial infection of the LF or LFC in an amount sufficient for combinative therapeutic effectiveness and one or more antibiotics in antibiotic-resistant microorganisms that are not susceptible to the direct microbicidal/growth inhibitory effects of LF or LFC.

For concurrent administration with antibiotics, the LF or LFC is to be administered in an amount effective to increase the antibiotic susceptibility of an antibiotic-resistant microbial species involved in the infection, or to potentiate the effects of the antibiotic. The LF or LFC is also to be administered in an amount affective to reverse the antibiotic resistance to antibiotic-resistant microbial species involved in the infection. The LF or LFC and the antibiotics each is to be administered in amounts that would be sufficient for therapeutic effectiveness upon administration alone or is to be administered in less than therapeutic amounts.

Another aspect of the invention provides a treatment of antibiotic-resistant microbial infections with LF or LFC and one or more antibiotic, in synergistically amounts.

In addition, the invention provides a method of killing or inhibiting growth of antibiotic-resistant microorganism comprising contacting the microorganism with the LF or LFC alone, or in combination with another antimicrobial agent. This method can be practiced in a variety of *in vitro* uses such as use in food preparation, to decontaminate fluids and surfaces or to sterilize surgical and other medical equipment and implantable devices, including prosthetic joints. These methods can correspondingly be used for *in situ* sterilization of indwelling invasive devices such as intravenous lines and catheters, which are often foci of infection, or for sterilization of *in vitro* tissue culture media.

In accordance with the present invention there is provided a method for the prevention and/or treatment of infections caused by antibiotic-resistant microorganisms or a surface, comprising treating a surface with a efficient amount of LF or LFC alone or in combination with an antibiotic, wherein the amount of LF or LFC is effective to substantially reverse resistance of the antibiotic-resistant microorganisms.

One aspect of the invention provides a method of treating antibiotic-resistant bacteria by affecting directly exoprotein gene expression and secretion by the LF or LFC alone or in combination with one or more antibiotic.

Another aspect of the invention is to provide a method for the desinfection and/or prevention of infection caused by antibiotic-resistanc microorganisms.

In accordance with the present invention there is also provided a composition for the prevention and/or treatment of infections caused by antibiotic-resistant microorganisms of a surface or a subject, comprising an efficient amount of LF or LFC in combination with an antibiotic, selected from ampicillin, cefazolin, neomycin, and novobiocin and penicillin in association with a acceptable carrier, wherein the amount of LF or LFC is effective to substantially reverse resistance of the antibiotic-resistant microorganisms.

In accordance with the present invention, there is provided the use of LF or LFC, ar a composition as defined above for treating, desinfecting and/or preventing infections caused by antibiotic-resistant microorganisms, or for the manufacture of medicament for the previously cited use.

The antibiotic-resistant microorganism may be selected from the group consisting of *Staphylococcus, Streptococcus, Micrococcus, Peptococcus, Peptostreptococcus, Enterococcus, Bacillus, Clostridium, Lactobacillus, Listeria, Erysipelothrix, Propionibacterium, Eubacterium, Corynobacterium, Mycoplasma, Ureaplasma, Streptomyces, Haemophilus, Neisseria, Eikenella, Moraxellus, Actinobacillus, Pasteurella, Bacteroides, Fusomicroorganism, Prevotella, Porphyromonas, Veillonella*, *Treponema, Mitsuokella, Capnocytophaga*, *Campylobacter, Klebsiella, Shigella, Proteus*, and *Vibriae.*

The antibiotics may be selected from the group consisting of aminoglycosides, vancomycin, rifampin, lincomycin, chloramphenicol, and the fluoroquinol, penicillin, beta-lactams, amoxicillin, ampicillin, azlocillin, carbenicillin, mezlocillin, nafcillin, oxacillin, piperacillin, ticarcillin, ceftazidime, ceftizoxime, ceftriaxone, cefuroxime, cephalexin, cephalothin, imipenen, aztreonam, gentamicin, netilmicin, tobramycin, tetracyclines, sulfonamides, macrolides, erythromycin, clarithromycin, azithromycin, polymyxin B, ceftiofure, cefazolin, cephapirin, and clindamycin.

In accordance with the present invention, there is provided a composition to counteract the development of antibiotic resistant bacteria strains in subject or on surface with an efficient amount of LF of LFC alone or in combination with antibiotic, wherein the amount of LF or LFC affect induction of resistant gene.

For the purpose of the present invention the following terms are defined below.

The term "surface" is intended to mean any surfaces including, without limitation, a wall, a floor, a ceiling, a medical device, a medical furniture, a surgical device, a prosthesis, an orthesis, a biological fluid delivery container, (e.g. blood bag, ophtalmic drops bottle), a food processing device, a food collecting device and a tube.

The term "subject" is intended to mean a human, an animal, or a plant.

The term "effective amount" is intended to mean, when used in combination with an antibiotic and/or an antimicrobial agent against antibiotic resistant microorganisms, with respect to the lactoferrin or lactoferricin, an amount effective to increase the susceptibility of the microorganism to the antibiotic and/or the antimicrobial agent, and with respect to an antibiotic or an other antimicrobial agent means at least an amount of the antibiotic or the antimicrobial agent that produces microbicidal or growth inhibitory effects when administrated in conjunction with that amount of lactoferrin or lactoferricin. Either the lactoferrin or lactoferricin or the antibiotic or other antimicrobial agents, or both, may be administered in an amount below the level required for monotherapeutic effectiveness against an antibiotic-resistant microorganisms.

The term "Pharmaceutically acceptable carrier" is intended to mean any carrier suitable for administration to a subject by any routes of administration, such as intravenous, intramammary, subcutaneous, intraperitoneal, topical, intraocular, intratracheal, transpulmonary, or transdermal route. Such carriers include, without limitation, an aqueous medium, a lipidic medium, an aerosolized solution, a nebulized drugs, an irrigation fluid, a washing solution (for, e.g. washing or wounds), a physiological solution (e.g. 0.9 % saline solution, ear drops, ophtalmic drops, citrate buffered saline, phosphate buffered saline), a long lasting delivery system (e.g. liposomes), a biologic fluid (e.g. blood, serum, plasma), a food mixture, a food liquid (e.g. milk, water, mineral water, gazeified water), a pharmaceutical acceptable diluent, or adjuvent.

The term "antimicrobial agent" is intended to mean any agent including, without limitation, an antibiotic, a bacteriocin, a lantibiotic, a disinfectant, a non-antibiotic growth inhibitory acceptable substance.

## BRIEF DESCRIPTION OF THE DRAWINGS

**FIG. 1**. illustrates growth in MHB of *S. aureus* ATCC 25923 in presence of bovine lactoferrin (LF) or lactoferricin (LFC) alone or in combination with penicillin G (PG);

**FIG. 2** illustrates growth in MHB of *S. aureus* PC-1 in presence of bovine lactoferrin (LF) or lactoferricin (LFC) alone or in combination with penicillin G (PG;

**FIG. 3** illustrates the effects of lactoferrin (LF) alone or in combination with erythromycin on growth of *S. aureus* SHY97-3906 after 18-h of incubation;

**FIG. 4** illustrates the effects of lactoferrin alone or in combination with neomycin on the growth of *Escherichia coli* SHY97-3923 after 18-h of incubation;

**FIG. 5** illustrates the effects of lactoferricin (LFC) alone or in combination with cefazolin or neomycin on the growth of *Escherichia coli* SHY97-3923;

**FIG. 6** illustrates the growth inhibitory effect of the lactoferrin alone or in combination with different concentrations of neomycin on Klebsiella pneumoniae SHY99-723;

**FIG. 7** illustrates the effects of the lactoferricin (LFC) alone or in combination with cefazolin or neomycin on Klebsiella pneumoniae SHY99-723;

**FIG. 8** illustrates transmission electron micrographs of thin sections of *S. aureus* ATCC 25923 growth on MHA plates and labelled with polycationic ferritin. Control untreated cells were obtained after culture on drug-free media (A). Cells were exposed to 0.0078 μg/ml of penicillin G (B) or 9.1 μg/ml of bovine lactoferrin (C) alone or in combination of the respective concentrations of both compounds (D);

**FIG. 9** illustrates transmission electron micrographs of thin sections of *S. aureus* SHY97-4320 grown on MHA plates and labelled with polycationic ferritin. Control untreated cells were obtained after culture on drug-free media (A). Cells were exposed to 8 μg/ml of penicillin G (B) or 1 mg/ml of bovine lactoferrin (C) alone or in combination of the respective concentrations of both compounds (D);

**FIG. 10** illustrates transmission electron micrographs of thin sections of *S. aureus* PC-1 grown during 4-h on MHB and labelled with polycationic ferritin. Control untreated cells were obtained after culture on drug-free media (A). Cells were exposed to 8 μg/ml of penicillin G (B) or 16 μg/ml of bovine lactoferricin (C) alone or in combination of the respective concentrations of both compounds (D). (Bar, 1 μm);

**FIG. 11** illustrates transmission electron micrographs of thin sections of *S. aureus* PC-1 grown during 4-h on MHB and labelled with polycationic ferritin. Control untreated cells were obtained after culture on drug-free media (A). Cells were exposed to 8 μg/ml of penicillin G (B) or 16 μg/ml of bovine lactoferricin (C) alone or combination of the respective concentrations of both compounds (D). (Bar, 0.5 μm);

**FIG. 12** shows transmission electron micrographs of thin sections of *S. aureus* SHY97-4320 after 4-h growth in MHB, labelled with polycationic ferritin and wheat germ agglutinin-gold. Cells were grown with 1 mg/ml of bovine lactoferrin in combination with penicillin G (8 μg/ml) (A, Bar = 1 μm; B, Bar = 0.25 μm). Control are shown in C (Bar = 0.25 μm);

**FIG. 13** shows β-lactamase activity measured as $\Delta OD_{486\ nm}$ /min in *S. aureus* strains SHY97-4320 and PC-1 after 4-h of incubation at 37°C with 8 μg/ml of penicillin G (PG) and 1 mg/ml of bovine lactoferrin (LF) alone or in combination. Values are means of three separated experiments;

**FIG. 14** shows β-lactamase activity of *S. aureus* strain PC-1 after 4- and 22-h of incubation with penicillin G (PG, 8 μg/ml) and bovine lactoferricin (LFC, 32 μg/ml or 64 μg/ml) alone or in combination. Values are means of three separated experiments;

**FIG. 15** shows β-lactamase activity measured as $\Delta OD_{486\ nm}$ /min in *S. aureus* strains SHY97-4320 (A) and PC-1 (B) after 4-h and 22-h of incubation at 37°C with 8 μg/ml of penicillin G (PG) and 1 mg/ml of human lactoferrin (LF) alone or in combination (PG + LF). Values are means of three separated experiments;

**FIG. 16** shows β-lactamase activity measured as $\Delta OD_{486\ nm}$ /min in *S. aureus* strains SHY97-4320 after 30 and 60 min pre-incubation with 1 mg/ml bovine lactoferrin and exposed to 8 μg/ml of with penicillin G (PG) during 4-h of incubation at 37°C. Values are means of three separated experiments; and

**FIG. 17** shows SDS-PAGE of whole cell proteins of *S. aureus* SHY97-4320 after 4-h of growth on MHB (line 1), MHB + 8 μg/ml of penicillin G (line 2), MHB + 1 mg/ml of lactoferrin (line 3) and MHB + combination of the same concentrations of both compounds (line 4). Molecular mass markers are indicated on the left.

## DETAILED DESCRIPTION OF THE INVENTION

The present invention relates to the uses of lactoferrin (LF) or lactoferricin (LFC) as antimicrobial agents alone or in combination with antibiotic to treat infections caused by antibiotic-resistant microorganism. The methods and materials described in the invention are new and were not known from those working in the art. The method and materials are used to treat subjects suffering from antibiotic-resistant microbial infections. Most particularly, the present invention relates to products for potentiation of the clinical efficacy of antibiotics, both to treat and prevent infectious diseases caused by pathogenic antibiotic-resistant microorganisms. The products of the present invention contain lactoferrin and its metabolized form the lactoferricin, and show remarkable potentiating effect on the efficacy of antibiotics.

Numerous additional aspects and advantages of the invention will become apparent to those skilled in the art upon consideration of the following detailed description of the invention that describes presently preferred embodiments thereof.

The present invention also relates to methods for the prevention and treatment of microbial diseases in mammals, including human being, and plant, and disinfecting of surgical devices, prosthesis, or food processing apparatus.

Antibiotic-resistant microbial infection, as used herein, encompasses conditions associated with or resulting from antibiotic-resistant microbial infections. These conditions include antibiotic-resistant sepsis and one or more of the conditions associated therewith, including bacteremia, fever, hypertension, shock, metabolic acidosis, disseminated intravascular coagulation and related clotting disorders, anemia, thrombocytopenia, leukopenia, adult respiratory distress and related pulmonary disorders, renal failure and related renal disorders, hepatobiliary disease and central nervous system disorders, and mastitis. These conditions also include translocation of antibiotic-resistant microorganism from digestive tube and concomitant release of endotoxin. Antibiotic-resistant microorganism from the following species: *Staphylococcus, Streptococcus, Micrococcus, Peptococcus, Peptostreptococcus, Enterococcus, Bacillus, Clostridium, Lactobacillus, Listeria, Erysipelothrix, Propionibacterium, Eubacterium, Corynobacterium, Mycoplasma, Ureaplasma, Streptomyces, Haemophilus, Neisseria, Eikenella, Moraxellus, Actinobacillus, Pasteurella, Bacteroides, Fusomicroorganism, Prevotella, Poxphyromonas, Veillonella, Treponema, Mitsuokella, Capnocytophaga, Campylobacter, Klebsiella, Shigella, Proteus, Vibriae.*

Among antibiotic-resistant microorganism, the most important microbial species involved in sepsis are Staphylococci, Streptococci, and Enterococci, but any antibiotic-resistant microorganism can be involved.

According to one aspect of the present invention, LF or LFC alone, in an amount sufficient for therapeutic efficiency, can be administered to a subject suffering from infection involving LF- or LFC-susceptible microorganism, or used as disinfectant of surgical and food processing devices. When used to describe administration of LF or LFC alone, the term "amount sufficient for therapeutic effectiveness" means an amount of LF or LFC that provides microbicidal or growth inhibitory effects when administered as a therapeutic dose. The invention utilized any of forms of LF or LFC known of the art including purified from neutrophil, or milk, recombinant LF or LFC, fragments of LF or LFC, LF or LFC variants, and LF or LFC derived-peptides. This aspect of the invention is based on the discovery that LF or LFC have direct microbicidal or growth inhibitory activity against some antibiotic-resistant microorganisms. LF or LFC are also shown herein to have direct microbicidal or growth inhibitory effects on different growth phases of different antibiotic-resistant microorganisms. Growth phases are the L-phase (Latent growth phase) and the S-phase (Exponential growth phase). LF or LFC are also expected to exert direct microbicidal/growth inhibitory effects on the cell wall-less *Mycoplasma* and *Ureaplasma,* miscroorganisms involved clinically in respiratory and urogenital infections. In addition, more than 100 subspecies of *Mycoplasma* constitute major contaminant of *in vitro* tissue cultures.

Another aspect of the invention is that LF or LFC can act through different mechanisms on microorganisms, as on the cell wall of both gram-positive and gram-negative microorganism. LF or LFC can bind surface receptors (e.g. heparin-binding like receptors, fibronectin-binding like receptors, protein A binding like receptors, or antibody binding like receptors, lipopolysaccharide-binding proteins) on the cell wall than inhibits attachment to mammalian cells. If LF or LFC are allowed to reach inside the inner cytoplasmic membrane, the amphipathic nature of LF or LFC may allow it to penetrate the cytoplasmic membrane and exert a microbicidal effect. Thus, agents that act on or disrupt the cell walls of microorganism such as antibiotics, detergents or surfactants, anti-peptidoglycan antibodies, anti-lipoteichoic acid antibodies and lysosyme, may potentiate the activity LF or LFC by allowing access to the inner cytoplasmic membrane. If LF or LFC enter further inside the cells, the mechanism of action of LF or LFC may be by inhibition of transcription of plasmid or gene responsible of the resistance carried by antibiotic-resistant microorganism.

LF or LFC can be used also to treat or prevent microbial infections caused by antibiotic-resistant microorganisms by concurrent administration of LF or LFC in an amount sufficient for combinative therapeutic effectiveness and one or more antibiotics in amounts sufficient for combinative therapeutic effectiveness. This aspect of the invention contemplate concurrent administration of LF or LFC with any antibiotic or combinations of antibiotics, including beta-lactam antibiotics, with or without beta-lactamase inhibitors, aminoglycosides, tetracyclines, sulfonamides and trimethoprim, vancomycin, macrolides, fluoroquinolones and quinolones, polymyxins and other antibiotics.

This characteristic of the invention is based on the discovery that administration of LF or LFC improves the therapeutic effectiveness of antibiotics, e.g. by providing benefits in reduction of cost of antibiotic therapy and/or reduction of risk of toxic responses to antibiotics. LF or LFC are shown herein to lower the minimum concentration of antibiotics needed to inhibit *in vitro* growth of antibiotic-resistant microorganisms from 0 to 24 hours of culture. The microbicidal or growth inhibitory effect of LF or LFC can be direct or indirect. This aspect of the invention is directly linked to the additional discovery that administration of LF or LFC can reverse the antibiotic resistance of antibiotic-resistant microorganism. LF or LFC shown herein to reduce the minimum inhibitory concentration of antibiotics from a level within the clinically resistant range to a level within the clinically susceptible range. LF or LFC have then proved to convert normally antibiotic-resistant microorganism into antibiotic-susceptible microorganism.

Since LF or LFC are found in a large part of human nutriments without triggering immune response after oral absorption, the products of the invention can be used as food preservatives. LF or LFC can be utilized when mixed with foods, e.g., supplemented with milk, yogurt, skim milk powder, lactic acid microorganism fermented milk, chocolates, tablet sweets, powdered beverages, and any other food in which LF or LFC can be added to aliments as preservative. LF or LFC can be used also in combination with other food preservatives, colorants, and excipients. The invention include dilution of LF or LFC in water or other aqueous solution, natural or synthetic lipidic media, each one containing different concentration and combination of salts or glucidic products. Such combination of LF. or LFC alone or with other preservatives contain an effective amount of the active compound together with a suitable amount of carrier so as to provide the form for proper administration to the host.

In one of most preferred embodiments of the invention, minimal inhibitory concentrations is 1 mg/ml for LF and 12.5 μg/ml for LFC.

One of the ways for administrating LF or LFC is the oral one. The administration of LF or LFC is preferably accomplished with a pharmaceutical composition comprising the LF or LFC and a pharmaceutical acceptable diluent, adjuvant, or carrier. The LF or LFC may be administered without or in conjunction with known surfactants, other chemotherapeutic agents or additional known antimicrobial agents.

According to the aspect of effective synergy of the invention, or potentiating upon concurrent administration of LF or LFC with one or more antibiotics can be obtained in a number of ways. LF or LFC may convert antibiotic-resistant microorganisms into antibiotic-susceptible microorganisms or otherwise improve the antibiotic susceptibility of those microorganisms. Conversely, LF or LFC can potentiate antibiotics such as an antibiotic that acts on the cell wall or cell membrane of microorganism may convert LF-or LFC-resistant microorganisms into LF- or LFC-susceptible microorganisms. Alternatively, LF or LFC and antibiotic may both co-potentiate the other agent's activity. The LF or LFC and antibiotic may have a therapeutic effect when both are given in doses below the amounts sufficient for therapeutic effectiveness.

Either- LF or LFC, or the antibiotics may be administered systemically or topically. Systemic routes of administration include oral, intravenous, intramuscular or subcutaneous injection, intraocular or retrobulbar, intrathecal, intraperitoneal, intrapulmonary by using aerosolized or nebulized drug, or transfermal. Topical route includes administration in the form of salves, ophthalmic drops, eardrops, or irrigation fluids. LF or LFC alone or in combination with antibiotics can be delivered also in different delivery systems, long lasting or rapidly degraded.

An advantage provided by the present invention is the ability to provide effective treatment of antibiotic-resistant microorganism by improving the therapeutic effectiveness of antibiotics against these microorganism. Because their systemic toxicity or prohibitive cost limits the use of some antibiotics, lowering the concentration of antibiotic required for therapeutic effectiveness reduces toxicity and/or cost of treatment, and thus allows wider use of antibiotic.

Among antibiotics that can be used alone or in different combination with other antibiotics and/or with LF or LFC are vancomycin, rifampin, lincomycin, chloramphenicol, and the fluoroquinol, penicillin, beta-lactams, amoxicillin, ampicillin, azlocillin, carbenicillin, mezlocillin, nafcillin, oxacillin, piperacillin, ticarcillin, ceftazidime, ceftizoxime, ceftriaxone, cefuroxime, cephalexin, cephalothin, imipenen, aztreonam, aminoglycosides, gentamicin, netilmicin, tobramycin, neomycin, tetracyclines, sulfonamides, macrolides, erythromycin, clarithromycin, azithromycin, polymyxin B, and clindamycin.

Biologically active fragments of LF or LFC include biologically active molecules that have the same or similar amino acid sequences as a natural human or bovine LF or LFC. Biologically active variants of LF or LFC include also but are not limited to recombinant hybrid fusion proteins, comprising LF or LFC analogs or biologically active fragments thereof and at least a portion of at least one other polypeptide, and polymeric forms of LF or LFC variants. Fusion protein forms can be designed in manner to facilitate purification processes. Biologically active analogs of LF or LFC include but are not limited to LF or LFC wherein one or more amino acid residues have been replaced by a different amino acid.

The invention also provides improved method of *in vitro* treatment of devices, work places, rooms, and liquids contaminated with antibiotic-resistant microorganism by contacting the microorganism with LF or LFC alone, or in combination with one or more antimicrobial products (e.g. antibiotics, detergents). The quantities of LF or LFC and antimicrobial products used are quantities that are separately sufficient for microbicidal/growth inhibitory effects, or quantities sufficient to have additive or synergistic microbicidal/growth inhibitory effects. These methods can be used in a variety of *in vitro* applications including sterilization of surgical and other medical equipment and implantable devices, including prosthetic joints. These methods can also be used for *in situ* sterilization of indwelling invasive devices such as intravenous lines and catheters, which are often foci of infection. The present invention concerns particularly treatment and prevention of human and animal microbial infections by antibiotic-resistant microorganisms.

Therapeutic effectiveness is based on a successful clinical outcome, and does not require that the antimicrobial agent or agents kill 100% of the organisms involved in the infection. Success depends on achieving a level of antimicrobial activity at the site of infection that is sufficient to inhibit the microorganism in a manner that tips the balance in favor of the host. When host defenses are maximally effective, the antimicrobial effect required may be minimal. Reducing organism load by even one loge (a factor of 10) may permit the host's own defenses to control the infection. In addition, augmenting an early microbial/bacteriostatic effect can be more important than long-term microbicidal/bacteriostatic effect. These early events are a significant and critical part of therapeutic success, because they allow time for host defense mechanisms to activate. Increasing the microbial rate may be particularly important for infections such as meningitis, bone or joint infections.

The present invention will be more readily understood by referring to the following examples that are given to illustrate the invention rather than to limit its scope.

## EXAMPLE I

### Determination of the minimal inhibitory concentration (MIC) of antibiotics

### Antimicrobial agents

Bovine apo-lactoferrin, novobiocin (quinolone-like antibiotic) and the macrolide erythromycin were purchased from Sigma Chemicals (St-Louis, Mo.). Penicillin G, ampicillin, cefazolin and neomycin were purchased from Novopharm Limited (Toronto, ON, Canada). Bovine LF (Besnier, California USA) was stored at -20°C at a concentration of 100 mg/ml in water. Lactoferricin was isolated from bovine LF (Besnier, California USA) according to the procedure described by Dionysius. and Milne (1997, J. Dairy Sci. **80**:667-674)) and it was kept at -20°C until use. The isolated peptide was sent at the Biotechnology Research Institute (Montreal, QC, Canada) for amino acids sequencing which confirmed that it was LFC. Antibiotics stocks were always freshly prepared and diluted to the desired concentration in Mueller Hinton agar plates (MHA) or broth (MHB). A panel of discs of antibiotics (Becton Dickinson Microbiology Systems, Cockeysville MD), including ampicillin (10 μg), penicillin (10 u), cephalotin (30 μg), neomycin (30 μg), tetracyclin (30 μg), oxacillin (1 μg), erythromycin (15 μg), sulfamethoxazol (23.7 μg) / trimethoprim (1.25 μg), novobiocin (30 μg), penicillin (10 u) / novobiocin (30 μg), pirlimycin (2 μg), gentamycin (10 μg), spectinomycin (100 μg) was used in quality control assays.

### Bacterial strains and growth condition

*Staphylococcus* aureus strains ATCC 25923 and 6538 and *Escherichia coli* ATCC 25922 were obtained from the American Type Culture Collection. Eight bovine mastitis clinical isolates of S. *aureus* (SHY97-3906, -3923, -4085 -4242, -4320 and -4343, RFT-1 and RFT-5) and one isolate of *E. coli* SHY97-3923-2 and one isolate of *Klebsiella pneumoniae* SHY99-723-1 were kindly provided by the Laboratoire Provincial de Pathologie Animale of St-Hyacinthe and of Rock-Forest (Quebec, Canada), respectively. Nine β-lactam antibiotics resistant *S. aureus* strains (PC-1, NCTC 9789, 2076, 22260, ST79/741, 3804, RN9, FAR8, and FAR10) were obtained from Vanderbilt University School of Medicine (Nashville, Tennessee, USA). All the culture media were from Difco Laboratories (Detroit, MI). Bacteria from frozen stock at -80°C were streaked onto tryptic soy agar plates suplemented with 5% of defibrinated sheep blood (Quelab, Montreal, QC, Canada) or Brain Heart infusion agar plates. Plates were then incubated for 16 to 24 h at 37°C. For most experiments, the strains were subcultured onto Mueller Hinton agar plates (MHA) or broth (MHB) for an additional 16-20 h. Aqueous solutions of the tested products were added by filtration through a sterile filter assembly (pore size 0.2 μm, Fisher, Ottawa, Ontario).

The MICs were determined by both macrodilution (1 ml/tube) and microdilution (100 μl/well) in sterile 96 well microtiter plates (International Nunc, Napierville, Il) techniques in three separate experiments according to the National Committee for Clinical Laboratory Standards (1992 and 1999) in three separate experiments. Serial 2-fold dilutions in MHB of LF, LFC, antibiotic or combination of LF or LFC with antibiotic were inoculated with an overnight culture at a final inoculum of $10^6$ cfu/ml in MHB. Effect of LF combination with antibiotic on MICs were determined by adding 0.5 mg or 1 mg/ml of LF to each antibiotic dilution. The MIC was defined as the lowest concentration of drug (highest dilution) that caused a complete inhibition of bacterial growth after an incubation of 18 h. Effect of LF or LFC combination with antibiotic on MIC was also determined by checkerboard method using 96 well microtiter plates. Each antibiotic dilution (50 μl) was serially added to the wells in vertical rows starting from the top (lower dilution) to the bottom. Lactoferrin or LFC (50 μl) were added to 50 μl of each antibiotic dilution and serially diluted starting at the left (lower dilution) to the right. After inoculation, the final concentration of antibiotic from top to bottom were 32 to 8, 2 to 0.5, 0.5 to 0.125 μg/ml for penicillin, cefazolin and neomycin, respectively. From left to right, the concentration of LF or LFC were 25 to 0.0244 mg/ml or 256 to 0.5 μg/ml respectively in a final volume of 100 μl/well. The microplates were incubated at 37°C for 24 h. Bacterial growth was measured optically at 540 nm using Spectra Max 250 Microplate Spectrophotometer System of Molecular Device (Fisher Scientific, Ottawa, Canada). Tubes were incubated at 37°C for 18 h and bacterial growth was measured by visual examination and optically at 540 nm using a spectrophotometer (Philips PU 8800; Pye Unican Ltd, Cambridge, UK). The fractional inhibitory concentration (FIC) was calculated as described by Eliopoulos and Moellering (1991).

$$FIC_{\text{antibiotic A}} = MIC_{\text{antibiotic A}} \text{ (in the presence of antibiotic B) } / \ MIC_{\text{antibiotic A}} \text{ alone.}$$

$$\text{FIC Index} = \text{FIC}_{\text{antibiotic A}} + \text{FIC}_{\text{antibiotic B}}$$

The effects of the antibiotics were considered to be synergistic or indifferent when FIC Index was < 1 or > 1, respectively.

For quality control, disk diffusion susceptibility tests, as recommended by the National Committee for Clinical Laboratory Standard, were performed on all strains using standard disk of various antibiotics. After 18 h of incubation at 37°C, the diameter of the zone of complete inhibition of bacterial growth around each disk was measured. Isolates were categorized as sensitive or resistant for a given antibiotic using the recommended interpretative guidelines of the manufacturer.

The MICs of penicillin G alone or in combinations with 0.5 or 1 mg/ml LF obtained for several *S. aureus* strains are given in Table 1. Except for strain ATCC 25923 and the field isolate strain SHY97-3923, all the other strains including the two clinical mastitis strains (SHY97-3906 and SHY97-4320) were resistant to penicillin G with MICs of 0.5 to > 128 μg/ml. Standard disk diffusion and a cefinase test confirmed the resistance to penicillin and ampicillin and the production of β-lactamase in these strains. Lactoferrin alone demonstrated weak inhibitory activity against these strains with MICs greater than 25 mg/ml. The MICs of LFC were 128 μg/ml for ATCC 25923 and 256 μg/ml for the others strains. Combination of 0.5 mg/ml of LF to penicillin increased the inhibitory activity of penicillin by two-fold in all tested strains except for ATCC 25923 and SHY97-3906 which needed a LF concentration of 1 mg/ml (Table 1).

Examination of FIC index indicate synergistic effects between LF and penicillin, novobiocin and erythromycin. Combination of LF to penicillin increased the inhibitory activity of penicillin by 2 and ≥ 2-fold in strain ATCC 25923 and PC-1, respectively. This increase was four fold in strains SHY97-4320 and SHY97-3906 (Table 2). The inhibitory activity of LF was increased by 16 to 64 folds by penicillin G (Table 2). Combination of LF to novobiocin increased the inhibitory activity of penicillin by 2 to 4 fold in 7/10 (70%) of *S. aureus* strains tested whereas activity of erythromycin was increased in the presence of LF by 2 to 16 fold in the same percentage of *S. aureus.*

Lactoferrin is an important iron-chelator protein. In this experiment, we show that LF has a low antibacterial activity against *S. aureus*. This bacteria is able to grow in the presence of extremely low (0.04 μM) iron concentration. We observed that bovine LF saturated to 16.2% of iron also demonstrated a growth inhibitory activity. Accordingly, addition of 5 μM of $FeCl_3$ did not affect LF growth inhibitory activity. Therefore, it appears that the antibacterial activity of LF against *S. aureus* is not only due to its iron chelating property.

Combination of antibiotics are used in the treatment of infectious diseases to provide a broad spectrum of coverage, to reduce the emergence of resistant strains and drug toxicity and finally to produce synergistic or additive effects between antibiotics. We found that combination of penicillin G, novobiocin or erythromycin with relatively low concentration of bovine LF lead to the increase of antibacterial activity of these antibiotics against most tested strains. In the presence of relative low concentration of bovine LF in the media, the growth of Staphylococci strains isolated from bovine clinical mastitis is inhibited by penicillin G to a greater extend. This finding indicated that combination of penicillin and LF act synergistically against these strains. In general, the FIC Index of penicillin and erythromycin in the presence of LF were lower in β-lactamase producing strains than in non-producing β-lactamase strains. This suggest that LF can reduce antibiotic resistance.

## EXAMPLE II

**Effect of bovine lactoferrin / lactoferricin and antibiotics on bacterial growth.**

The effect of LF or LFC at concentration sub-MICs alone or in combination with sub-MICs of antibiotics on bacterial growth rate of *S. aureus*, *E. coli* and *K. pneumoniae* was determined by monitoring bacterial cultures in MHB with the $O.D._{600\ nm}$ or by the count of colony forming unit per ml (cfu/ml). A volume of 2.5 ml of overnight cultures in MHB adjusted to 0.5 - 1 McFarland standard in saline were used to inoculate a final volume of 25 ml of fresh MHB containing the desired concentration of tested compounds. All flasks were then incubated at 37°C with agitation (200 rpm) for 9 h. Aliquots were removed every hour to determine the culture turbidity using the spectrophotometer Philips PU 8800 (Pye Unican Ltd, Cambridge, UK). The combined antibiotic effect on bacterial growth was also determined using concentrations of LF in the presence of different concentrations of antibiotics. Briefly, a volume of 3 ml of fresh MHB containing desired concentration of drugs was adjusted to an optical density of 0.4 at 540 nm after an overnight culture. The culture was then incubated at 37°C with agitation (200 rpm). After incubation, aliquots were removed to determine the culture turbidity ($OD_{540nm}$) or cfu/ml. To determine the influence of the level of iron on of the growth inhibition by the test compound, 5 μM of $FeCl_3$ were added to the media.

The effects of penicillin G in combination with LF or LFC on growth rate obtained with *S. aureus* strain ATCC 25923 and constitutive β-lactamase producing strain PC-1 are presented in Fig. 1 and 2. Sub-MIC of penicillin G alone did not significantly affect growth rate of PC-1. In strain ATCC 25923, LF, and in strain PC-1, LF and LFC alone delayed growth. When 1 mg/ml LF was used in combination with sub-MIC of penicillin G (1/4 to 1/16 MIC), important growth rate reductions were observed (P < 0.01). Complete growth inhibition was obtained when penicillin G was combined with 32 μg/ml (1/8 MIC for strain ATCC 25923) or 64 μg/ml (1/8 MIC for PC-1) of LFC. For the non-producing β-lactamase clinical *S. aureus* strains SHY97-4242 and RFT-5, penicillin G also reduced the growth of these strains (P < 0.001). The growth inhibitory activity of penicillin was enhanced by the presence of $L_F$ (P < 0.01). Addition of iron to the media did not affect the growth inhibition induced by combination of penicillin to LF.

The effect of erythromycin, novobiocin, LF alone and their combination were evaluated on growth of *S. aureus* strains ATCC 25923, SHY97-3906, -4242 and RFT-5 after 4, 8 and 18-h of incubation. Alone, sub-MIC (9.1, 36.6, 250 or 500 μg/ml) of LF did not affect the growth of these microorganisms except for strain SHY97-3906 (P > 0.5). Strain SHY97-3906 was affected by combination of erythromycin and LF after 18-h of incubation (Fig. 3).

The effects of combinations of LF or LFC with neomycin also were evaluated on the growth of the clinical isolates of *E. coli,* SHY97-3923 and K. *pneumoniae* SHY99-723. For the *E. coli* strain, the effect of neomycin on bacterial growth was significantly enhanced when 1 mg/ml of LF (Fig. 4) or 16 μg/ml LFC (1/16 MIC; Fig 5) was added respectively to the media. Similar results were found for the strain of *K. pneumoniae* tested. The bacterial growth rate of this strain with neomycin was reduced by 2 times when 0.5 mg/ml of LF (Fig. 6) or 16 μg/ml LFC (1/16 MIC; Fig. 7) was added respectively to the media. The synergy with neomycin was stronger with LFC than with LF.

Alone, the β-lactam antibiotic cefazolin (0.5 :g) was not able to reduce growth of *E. coli* SHY97-3923 (Fig 5). However, it synergysed with LFC and completely inhibit bacterial growth.

## EXAMPLE III

### Effect of bovine lactoferrin / lactoferricin and antibiotics on bacterial cell morphology.

Bacterial cells were grown overnight on MHA or MHB containing sub-MICs of penicillin G with or without LF or LFC. Microorganisms were prepared for transmission electron microscopy by fixation with glutaraldehyde followed by ferritin labelling. This method allows good preservation of capsular material. Briefly, bacterial cells grown in the presence or absence of antibiotics were fixed in cacodylate buffer (0.1 M, pH 7.0) containing 5% (v/v) glutaraldehyde, for 2 h at 20°C. Fixed microorganims were suspended in cacodylate buffer and allowed to react with the polycationic ferritin (Sigma Chemicals, St-Louis, Mo; final concentration 1.0 mg/ml) for 30 min at 20°C. The reaction was slowed down by 10-fold dilution with buffer, and the microorganisms were centrifuged and washed three times in cacodylate buffer. Bacterial cells were then immobilized in 4% (w/v) agar, washed 5 times in cacodylate buffer and post-fixed with 2% (w/v) osmium tetroxide for 2 h. Washings were repeated as above, and the samples dehydrated in a graded series of acetone washes. Samples were then washed twice in propylene oxide and embedded in Spurr low-viscosity resin. Thin sections were post-stained with uranyl acetate and lead citrate and examined with an electron microscope (Philips 201) at an accelerating voltage of 60 kV.

In order to compare the effect of sub-MICs of penicillin G and LF alone or in combination on cell morphology, *S. aureus* SHY97-4320 and ATCC 25923 were collected after 4 and 18-h of incubation, respectively (Fig. 8 and 9). In strain ATCC 25923, sub-MIC of penicillin G (0.0078 μg/ml) induced formation of large symmetrically arranged pseudomulticellular staphylococci with two division planes and multiple cross walls (Fig 8B). Thicker septa with irregular aspect were also observed after exposure to sub-MIC of penicillin G. Lactoferrin at concentration of 9.1 μg/ml showed no obvious effect on *S. aureus* cells (Fig 8C). The effect of sub-MICs of penicillin G and LF in combination on the morphology of *S. aureus* were similar but not identical to that observed with penicillin G alone. Indeed, asymmetrically arranged pseudomulticellular staphylococci were formed, cross walls were thicker, irregular and some time non-existent (Fig. 8D). Irregular and lysing bacterial cells as well as cell wall fragments and cells with broken walls and debris were also observed with this treatment. In resistant strain SHY97-4320, penicillin G (8 μg/ml) alone had no visible effect (Fig 9B), but when it was combined with LF (1 mg/ml), morphology changes were similar to that observed in the susceptible strain (Fig. 9A to 9D). This suggests that LF can restore susceptibility of resistant strains to penicillin.

Bacterial cells of PC-1 (a constitutively high producing β-lactamase strain) were grown overnight in MHB containing 8 μg/ml of penicillin G, 16 μg/ml of LFC alone or in combination for 4-h at 37°C with agitation at 150 rpm. Bacteria cell morphology was evaluated by transmission electron microscopy after fixation and ferritin labelling method as previously described. Penicillin alone had no effects on morphology. Exposure to LF or LFC affected the shape and the size of *S. aureus.* A large percentage of lysed bacteria was observed with LFC which was enhanced in the presence of penicillin G (Fig. 10C and 10D, see arrows). In addition, LFC induced formation of mesosome structures arising from the septa and cell wall in *S. aureus* (Fig. 11). Again, this suggests that LF can restore susceptibility of resistant strains to penicillin.

To investigate the mechanism of action of LF in combination with penicillin G on cell division, transmission electron microscopy was also performed on thin section of a β-lactamase producing *S. aureus* SHY97-4320. Bacterial cells were grown in MHB containing 8 μg/ml of penicillin G, 1 mg/ml LF alone or in combination for 4-h at 37 °C with agitation at 150 rpm. Bacteria were harvested and incubated or not for 2-h in 0.02 M Tris (pH 7.4) containing 0.15 M NaCl, 0.5 mg/ml and 50 μl of wheat germ agglutinin (WGA) gold (Sigma Chemicals, St-Louis, Mo) for 2 h. Bacteria cell morphology was evaluated by transmission electron microscopy as previously described. Effects of treatments were evaluated and compared by "t" test. Groups of multiple undivided cell were observed after treatment of LF alone or in combination with penicillin (Fig. 12A). These results suggest that LF can affect staphylococcal cell division. The WGA has an affinity for N-acetyl-β-D-glucosamyl residues and N-acetyl-β-D-glucosamine oligomer. After treatment with LF, *S. aureus* cells were less covered ($P < 0.005$) with WGA-gold (Fig. 12 and Table 3). These results also suggest that LF affect formation or accessibility of N-acetyl-β-D-glucosamine, which is an important part of cell wall peptidoglycan.

**EXAMPLE IV**

**Effect of bovine lactoferrin / lacoferricin on. β-lactamase production**

The effects of sub-MICs of LF (1 mg/ml), LFC (32 and 64 ug/ml) alone or in combination with ampicillin (4ug/ml) or penicillin G (8 μg/ml) were evaluated on the β-lactamase activity of *S. aureus* strains SHY97-4320 and PC-1. The chromogenic cephalosporin nitrocefin (Becton Dickinson Microbiology, Cockeysville, MD) was used in a quantitative spectrophotometric assay. Bacterial cells were first exposed during 4 and 22-h to drugs in broth. The number of bacteria (cfu/ml) were determined and cells aliquots were centrifuged at each point in time. The pellets were suspended in 10 mM HEPES buffer (pH 7.4) to an $OD_{415\ nm}$ of 1 for PC-1 and 2 for SHY97-4320. Nitrocefin (100 μM) was added to 100 μl of cells in a final volume of 1 ml. Hydrolysis was measured at 486 nm on spectrophotometer. β-lactamase activity was expressed as ΔO.D.486nm/min and corrected for bacterial OD.

In strain SHY97-4320, no β-lactamase activity was shown in control and LF containing cultures (Fig. 13). In the culture containing 8-μg/ml penicillin G, a large increase in β-lactamase activity was observed. When LF was used in combination with penicillin G, a significant reduction of β-lactamase activity was observed ($P < 0.001$). In strain PC-1, LF moderately reduces β-lactamase activity ($P < 0.05$). However, in this strain, LFC demonstrated a strong ($P < 0.001$) inhibition of β-lactamase activity (Fig 14). Similar results were obtained with ampicillin. These results indicate that LF and LFC repress resistance to β-lactam antibiotics by inhibiting β-lactamase activity.

**EXAMPLE V**

**Effect of human lactoferrin on β-lactamase production**

Penicillin G was purchased from Novopharm Limited (Toronto, Ontario, Canada). Human LF (Sigma) was stored at -20°C at a concentration of 100 mg/ml in water. Antibiotic stock solutions were always freshly prepared and diluted to the desired concentration in Mueller Hinton broth (MHB) medium (Difco Laboratories, Detroit, MI). β-Lactamase activity was measured using a quantitative spectrophotometric assay with the chromogenic cephalosporin nitrocefin. Bacterial cells were first exposed to penicillin and/or LF during 4 and 22-h in broth. At each time point, the number of cfu/ml were determined and cells aliquots were centrifuged.

The pellets were suspended in 10 mM HEPES buffer (pH 7.4) to an $OD_{415\ nm}$ of 0.4 for PC-1 and 4 for strain SHY97-4320. Nitrocefin (100 μM) was added to 20 μl of cells in a final volume of 200μl. Hydrolysis was measured at 486 nm on a Spectra Max 250 Microplate Spectrophotometer System of Molecular Device (Fisher Scientific, Ottawa, Canada). β-Lactamase activity was expressed as ΔO.D.486nm/min and corrected for bacterial OD.

The effect of human LF and/or penicillin G on β-lactamase activity was evaluated in *S. aureus* strains SHY97-4320 and PC-1 (Fig. 15). In strain SHY97-4320, no β-lactamase activity was observed in control and LF (1mg/ml) containing cultures. In the culture containing 8-μg/ml penicillin G, β-lactamase activity was present. When 1 mg/ml of LF was added at the same time as penicillin G, an important reduction of the β-lactamase activity was observed ($P < 0.001$). In strains PC-1, a constitutive producing β-lactamase strain, human LF also reduced by 50% and 20% β-lactamase activity after incubation for 4 and 22 h, respectively.

**EXAMPLE VI**

**Effect of bovine lactoferrrin and antibiotic on protein profile and signal transduction.**

*Staphylococcus aureus* SHY97-4320 cells growth in MHB containing test compounds were suspended in electrophoresis sample buffer containing 2% sodium dodecyl sulfate (SDS) and 5% 2-mercaptoethanol to a final concentration of 0.1 g per ml. The samples were heated to 100°C for 5 min before being loaded for electrophoresis on a discontinuous 0.1% SDS-polyacrylamide gel (SDS PAGE) with 6% polyacrylamide stacking gel and 10 or 12% polyacrylamide running gel. Gels were run on a Mini-Protean® II apparatus (Bio Rad laboratories, Richmond, CA). Protein profiles were visualised by staining with coomassie brilliant blue R-250 or by silver staining.

The protein profile of whole bacterial cells of the B-lactamase producing *S. aureus* SHY97-4320 obtained on SDS-PAGE after electrophoresis was examined. Several proteins were expressed and major differences were observed in these proteins when cultures conditions were compared. Proteins of approximately 59, 42 and 27 kDa can be seen in the control and the culture containing penicillin G very clearly, but were absent in the culture with LF alone or in combination to penicillin G (Fig 17). The lack of the 27 to 59-kDa protein band suggest that LF probably inhibit the synthesis and/or secretion of these proteins. Lack of these proteins also can explain the synergistic effect between LF and penicillin and the restoration of susceptibility in resistant strains. Similar changes were observed in *S. aureus* strains PC-1, NCTC 9789 and ATCC 25923.

## EXAMPLE VII

### Effect of lactoferrin on signal transduction

As the β-lactamase system is the best-known signal transduction system in *S. aureus.* We tested the ability of LF to inhibit signal transduction in *S. aureus* SHY97-4320 which is an inducible beta-lactamase producing strain. Bacterial cells were first exposed during 30 or 60 min to LF in broth and were further treated with 8 μg/ml of penicillin G for an additional 4 h. The cfu/ml were determined, cells aliquots were centrifuged and pellets were suspended in 10 mM HEPES buffer (pH 7.4) as mentioned above. Nitrocefin (100 μM) was added to 20 μl of cells in a final volume of 200 μl. Hydrolysis was measured at 486 nm on a Spectra Max 250 Microplate Spectrophotometer System of Molecular Device (Fisher Scientific, Ottawa, Canada). β-Lactamase activity was expressed as ΔO.D.486nm/min and corrected for bacterial OD.

In *S. aureus,* the synthesis of β-lactamase is organised in a operon comprised of a repressor gene *(blaI)* and an antirepressor (*blaR1*) which regulate the β-lactamase gene *(blaZ).* Proteolysis of BlaI by BlaR1 was shown to allow the synthesis of β-lactamase. Lactoferrin completely block induction of β-lactamase when it was added 30 or 60 min before penicillin G (Fig 16). These results show that LF or LFC affect the induction and/or the synthesis of β-lactamase by interfering with either *BlaR1* or the entire function of the *bla* operon and therefore blocking the induction of beta-lactamase synthesis and secretion induced by penicillin through signal transduction.

## EXAMPLE VIII

### Effect of lactoferrin on bacterial gene expression

### Staphylococcal strains and media

A β-lactam resistant clinical isolate strain (*S. aureus* SHY97-4320) was used to study the effect of LF and/or penicillin G on gene expression. The strain was kept frozen at -80°C in MHB containing 7% of DMSO until used. All culture media were from Difco (Detroit, MI, USA). Bacteria from frozen stock were cultured in Mueller Hinton media for 16 to 18-h and subcultured onto Mueller Hinton broth (MHB). Aqueous solutions of the tested products were added by filtration through a sterile filter assembly (pore size 0.2 μm, Fisher, Ottawa, Ontario).

### Antibiotics and reagents

Penicillin G was purchased from Novopharm Limited (Toronto, Ontario, Canada). Bovine LF (Besnier, San Juan Capistrano, CA USA) was stored at -20°C at a concentration of 100 mg/ml in water. Antibiotic stock solutions were always freshly prepared and diluted to the desired concentration in MHB medium (Difco Laboratories, Detroit, MI). Standard powder of nitrocefin was used to evaluate β-lactamase activity. Restriction endonucleases were purchased from Amersham Pharmacia Biotech.

### Bacterial growth conditions

Fresh MHB containing or not 8 μg/ml of penicillin G and/or 1 mg/ml of bovine LF (2 x 2 factorial design) was adjusted to an optical density at 540 nm of 0.04 with an overnight culture of *S. aureus* and then incubated at 37°C with agitation (150 rpm). After 4 and 22-h of incubation, aliquots were removed to determine bacterial growth by viable count (colony forming unit per ml, cfu/ml), measuring the culture turbidity ($OD_{540nm}$) with a spectrophotometer Philips PU and to measure β-lactamase activity in bacterial cells. Following culture under the same condition, bacterial cells were collected for RNA extraction.

**Quantification of β-lactamase activity**

The pellets were suspended in 10 mM HEPES buffer (pH 7.4) to an $OD_{415\ nm}$ of 4. Nitrocefin (100 μM) was added to 100 μl of cells in a final volume of 1 ml. Hydrolysis was measured at 486 nm on a Spectra Max 250 Microplate Spectrophotometer System of Molecular Device (Fisher Scientific, Ottawa, Canada). β-lactamase activity was expressed as ΔO.D.486nm/min and corrected for bacterial OD.

**Primers**

The Oligonucleotides primers were synthesised by PE Applied Biosystem (Foster City, CA USA) for real time RT-PCR. Target DNA for amplification for real-time PCR was from the published sequence of the *BlaZ* gene.

**Extraction of total RNA**

Total bacterial RNA was prepared using the RNeasy minikit (Qiagen, Mississauga, ON, Canada). Washed bacteria were first treated with 50 μg/ml of lysostaphin (GramCracker, Ambion, Austin, TX) for 10 min at 37°C and RNA was purified according to the manufacturer's protocol. Contaminating DNA was removed from total RNA by using 1 U of Rnase-free Dnase I (Gibco Life Technologies, Grand Island, NY) in a 10 μl reaction mixture containing approximately 50 ng of total RNA per μl and 20mM Tris-HCl (pH 8.4), 2mM MgCl2 and 50mM KCl. The reaction mixture was incubated for 15 min at room temperature, and the Dnase I was inactivated by adding 1 μl of 25mM EDTA to the mixture and incubating for 10 min at 65°C before assessing quantity and purity. Amount of cellular RNA was then determined by measuring the $OD_{260\ nm}$ and $OD_{280\ nm}$.

***In Vitro* transcription of mRNA of the *BlaZ* gene from *S. aureus***

A 600 bp PCR fragment of the *BlaZ* gene of S. *aureus* PC-1 was purified using the QIAQUICK PCR Purification Kit and was cloned in a pGEM-T easy vector (Promega, Madison, WI) with a Rapid DNA Ligation Kit (Roche, Laval, QC, Canada) after amplification by RT-PCR. Briefly, RT-PCR was performed with the Ready-To-Go RT-PCR Beads (Amersham Pharmacia Biotech), resuspended in 45 μl Rnase-free water (Qiagen), with 1 μl of the reverse primer (10 μM 5'-TAGTCTTTTGGAACACCGTC-3', SEQ ID NO:1) and 2 μl of total RNA (25 ng/μl). Reverse transcription was conducted for 30 min at 42°C. Afterward, 1 μl of forward primer (10 μM, 5'-ACAGTTCACATGCCAAAGAG-3', SEQ ID NO:2) and 1 μl of reverse primer (10 μM) were added. PCR was performed at 94°C for 2 min followed by 30 cycles at 94°C for 30 s, 60°C for 30 s and 72°C for 45 s. The amplicon was quantified by spectrophotometry and on a 1.5% agarose gel.

**Ligation**

Ligation of the BLAZ amplicon with the pGem-T easy vector system was done as follow: 2 μl of buffer 2 (5x) was mixed with 1.5 μl of pGem-T easy vector (8 ng/μl) and 2.5 μl of *BlaZ* amplicon (10 ng/μl). The reaction volume was completed to 10 μl with Rnase-free water (Qiagen) before adding 10 μl of buffer 1 (2x) and 1 μl of T4 ligase (5 U/ml). The mixture was incubated 20 min at room temperature. A 2 μl aliquot was then used to transform *E. coli* HB101 competent cells (Gibco Life Technologies). After screening by PCR, plasmidic DNA was extracted from positive colonies, quantified by spectrophotometry and on a 0.8% agarose gel and used for *in vitro* transcription. The BLAZ amplicon portion of the new clones was also sequenced to confirm the BLAZ insert.

***In vitro* transcription**

The clone pGemT easy-BLAZ was first linearized with Sal I upstream of the T7 promoter before *in* vitro transcription of mRNA. Briefly, 600 ng of linear DNA was mixed with 1 μl of each dNTP (10 mM) and 2 μl of RNA T7 polymerase (15 U/μl), in a 20 μl reaction. The mixture was incubated at 37°C for 1 h before Dnase I treatment. mRNA was purified with a RNAeasy column (Qiagen) and quantified by spectrophotometry. The mRNA of BLAZ was then used for a standard curve in the Real-Time RT-PCR analysis.

**Detection of *S. aureus* BLAZ gene by Real-Time quantitative RT-PCR**

Real-Time fluorescence-based 5' nuclease PCR which is a widely accepted method for measuring gene expression levels was used to quantify β-lactamase *BlaZ* RNA by RT-PCR on ABI Prism™ 7700 (TaqMan®) sequence detector (PE Applied Biosystem, Foster City, CA). The forward primer, reverse.primer, and TaqMan probe for Real-Time RT-PCR amplification were designed with the PrimerExpress software (PE Applied Biosystem) to specifically amplify the. *S. aureus* BLAZ gene. Briefly, RNA (50 ng) was added to a 50 μl reaction mixture containing 25 μl of 2x RT-PCR One-Step Universal Master Mix, 1.25 μl of 40x MultiScribe and Rnase Inhibitor Mix, 4.5 μl of the forward primer (10 μM, 5'-AATTAAATTACTATTCGCCAAAGAGCA-3', SEQ ID NO:3), 4.5 μl of the reverse primer (10 μM, 5'-TGCTTAATTT-TCCATTTGCGATAA-3', SEQ ID NO:4), and 1.25 μl of TaqMan probe (10 μM, 6FAM-ACGCCTGCTGCTTTCGGCAA-GA-TANIRA, SEQ ID NO:5). Reverse transcription with the recombinant Moloney Murine Leukemia Virus (M-MuLV) Reverse Transcriptase (0.25 U/μl) was conducted at 48°C for 30 min. After initial activation of AmpliTaq Gold DNA polymerase (1.25 U/μl) at 95°C for 10 min, 40 PCR cycles of 95°C for 15 s and 60°C for 1 min were performed. The cycle threshold value ($C_T$), indicative of the quantity of target gene at which the fluorescence exceeds a pre-set threshold, was determined and compared to the $C_T$ of a standard curve containing known amounts of mRNA (1.1 x $10^2$ to 1.1 × $10^9$) from the BLAZ gene of *S. aureus* obtained by *in vitro* transcription. *Statistical* analysis were done on the LOG 10 of number of copies.

**Effect of lactoferrin on β-lactamase activity**

The effects of bovine LF and penicillin G on β-lactamase activity was identical to those observed previously (see example 4).

**Effect of lactoferrin on BlaZ transcription**

Lactoferrin reduced (P<0.001) by 35% the mRNA level in bacteria (Table 4). Additon of penicillin G to the growth medium induced a 28 fold increase (P<0.01) in the number of copies of the *BlaZ* mRNA per ml of culture. This increase was completely prevented by simultaneous addition of 1 mg/ml of LF (Table 4). These results indicate that LF reduces β-lactamase activity by inhibiting the expression *BlaZ* gene. Our data on protein profile of bacteria (see example 6) shows that the level of several proteins (especially secreted proteins) was reduced by LF. Here, we shows that LF results not only in a large decrease in *BlaZ* mRNA but also in a general reduction of RNA level indicating a general inhibition of gene expression. Therefore, LF can counteract all antibiotic resistance mechanisms that involve expression of genes.

**Table 1 Minimal inhibitory concentrations (MICs) of penicillin G alone or in combination with 0.5 mg/ml or 1 mg/ml of bovine lactoferrin (LF) as determined by macrodillution method against 13 *S. aureus* strains**

| *S. aureus* | β-lactamase type | MICs of penicillin G (μg/ml) | | |
|---|---|---|---|---|
| | | Alone | + 0.5 mg/ml LF | + 1 mg/ml LF |
| ATCC 25923 | - | 0.031 | 0.031 | 0.015 |
| SHY97-3923 | - | 0.015 | 0.007 | 0.007 |
| SHY97-3906 | + uncharacterised | 0.5 | 0.5 | 0.25 |
| SHY97-4320 | + uncharacterised | 64 | 32 | 32 |
| PC - 1 | + A constitutive | > 128 | 128 | 128 |
| NCTC | + A | > 128 | 128 | 128 |
| 2076 | + A | 0.5 | 0.25 | 0.25 |
| 22260 | + B | 32 | 16 | 16 |
| ST79/41 | + B | 32 | 16 | 16 |
| 3804[f] | + C | 128 | 64 | 64 |
| RN 9 | + C | 128 | 64 | 64 |
| FAR 8 | + D | 16 | 8 | 8 |
| FAR 10 | + D | 2 | 1 | 1 |

**Table 2 MICs and FIC index of penicillin in combination with bovine lactoferrin (LF) of MICs as determined by checkerboard macrodillution method against some *S. aureus* strains**

| Strain | MIC (μg/ml) | | | | FIC index |
|---|---|---|---|---|---|
| | LF | Decrease | Penicillin | Decrease | |
| *S. aureus* ATCC 25923 | 780 | 32 | 0.0156 | 2 | 0.53 (S) |
| *S. aureus* SHY97-3906 | 1560 | 16 | 0.125 | 4 | 0.31 (S) |
| *S. aureus* SHY97-4320 | 390 | 64 | 16 | 4 | 0.26 (S) |
| *S. aureus* PC-1 | 390 | 64 | 128 | ≥ 2 | ≤ 0.51 (S) |

The FIC index was calculated as described in the text, and its interpretation in the parentheses was: S, synergy (< 1).

**Table 3 Numbers of WGA-gold particles per μm of bacterial cell wall. *S. aureus* SHY97-4320 was treated with 8 μg/ml of penicillin G (PG), 1 mg/ml of lactoferrin (LF) alone or in combination**

| Treatment | Mean number of WG-gold particles ∀ SME |
|---|---|
| Control | 43.22 ∀ 4.14 |
| PG | 26.50 ∀ 5.29 |
| LF | 24.41 ∀ 3.20 |
| PG-LF | 26.09 ∀ 1.59 |

SME, standard error of means.

**Table 4 Effects of lactoferrin (1 mg/ml) and/or penicillin G on β-lactamase (BlaZ) gene expression in *S. aureus* strain SHY97-4320**

| Parameter | Treatment | | | |
|---|---|---|---|---|
| | Control | Penicillin G (PG) | Lactoferrin (LF) | PG-LF |
| RNA[1] (:g/ml) | 2.90 ± 0.26 | 3.22 ± 0.49 | 1.88 ± 0.68 | 1.89 ± 0.61 |
| *BlaZ*[2] | $1.1 \times 10^4$ | $22.9 \times 10^4$ | $1.4 \times 10^4$ | $0.6 \times 10^4$ |
| *BlaZ* total[3] | $6.1 \times 10^5$ | $174.9 \times 10^5$ | $5.6 \times 10^5$ | $2.2 \times 10^5$ |

[1] Adjusted for an $OD_{540nm}$ of 1.0.
[2] Number of copies of BlaZ gene mRNA/50 ng of mRNA.
[3] Total number of copies of BlaZ gene mRNA/ml of culture.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A composition for inhibiting β-lactamase in a microorganism comprising ampicillin, wherein said ampicillin is in concentration of about 4 μg/ml, and a protein selected from the group consisting of a lactoferrin, a lactoferricin or a fragment thereof, in association with an acceptable carrier.

2. A composition for inhibiting β-lactamase in a microorganism comprising cefazolin, wherein said cefazolin is in concentration of about 0.5 μg/ml, and a protein selected from the group consisting of a lactoferrin, a lactoferricin or a fragment thereof, in association with an acceptable carrier.

3. A composition for inhibiting β-lactamase in a microorganism comprising neomycin, wherein said neomycin is in concentration of between 0.125 μg/ml and 1 μg/ml, and a protein selected from the group consisting of a lactoferrin, a lartoferricin or a fragment thereof, in association with an acceptable carrier.

4. A composition for inhibiting β-lactamase in a microorganism comprising novobiocin, penicillin and a protein selected from the group consisting of a lactoferrin, a lactoferricin or a fragment thereof, in association with an acceptable carrier.

5. An in vitro method for disinfecting and/or preventing infection of a surface by β-lactamase producing microorganism, said method comprising treating said surface with an efficient amount of lactoferrin or lactoferricin.

6. The method of claim 5 further comprises treating with at least one antibiotic.

7. The method of claim 5, wherein said microorganism is selected from the group consisting of Staphylococcus, Streptococcus, Micrococcus, Peptococcus, Peptostreptococcus, Enterococcus, Bacillus, Clostridium, Lactobacillus, Listeria, Erysipelothrix, Propionibacterium, Eubacterium, Corynobacterium, Mycoplasma, Ureaplasma, Streptomyces, Haemophilus, Neisseria, Eikenella, Moraxellus, Actinobacillus, Pasteurella, Bacteroides, Fusomicroorganism, Prevotella, Porphyromonas, Veillonella, Treponema, Mitsuokella, Capnocytophaga, Campylobacter, Klebsiella, Shigella, Proteus and Vibriae.

8. The method of claim 6, wherein said antibiotic is selected from the group consisting of aminoglycosides, vancomycin, rifampin, lincomycin, chloramphenicol, and the fluoroquinol, penicillin, beta-lactams, amoxicillin, ampicillin, azlocillin, carbenicillin, mezlocillin, nafcillin, oxacillin, piperacillin, ticarcillin, ceftazidime, ceftizoxime, ceftriaxone, cefuroxime, cephalexin, cephalothin, imipenen, aztreonam, gentamicin, netilmicin, tobramycin, tetracyclines, sulfonamides, macrolides, erythromycin, clarithromycin, azithromycin, polymyxin B, ceftiofure, cefazoline, cephapirin, and clindamycin.

9. The method of claim 5, wherein said surface is selected from the group consisting of a wall, a floor, a ceiling, a medical device, a medical furniture, a surgical device, a prosthesis, an orthesis, a biological fluid delivery container, (e.g. blood bag, ophtalmic drops bottle), a food processing device, a food collecting device, and a tube.

10. Use of lactoferrin and/or lactoferricin in the manufacture of a medicament for prevention and/or treatment of infections caused by a β-lactamase producing microorganism.

11. The use of claim 10, wherein said lactoferrin and/or lactoferricin is in combination with at least one antibiotic.

12. The use of claim 10, wherein said microorganism is selected from the group consisting of Staphylococcus, Streptococcus, Micrococcus, Peptococcus, Peptostreptococcus, Enterococcus, Bacillus, Clostridium, Lactobacillus, Listeria, Erysipelothrix, Propionibacterium, Eubacterium, Corynobacterium, Mycoplasma, Ureaptasma, Streptomyces, Haemophilus, Neisseria, Eikenella, Moraxellus, Actinobacillus, Pasteurella, Bacteroides, Fusomicroorganism, Prevotella, Porphyromonas, Veillonella, Treponema, Mitsuokella, Capnocytophaga, Campylobacter, Klebsiella, Shigella, Proteus and Vibriae.

13. The use of claim 11, wherein said antibiotic is selected from the group consisting of aminoglycosides, vancomycin, rifampin, lincomycin, thloramphenicol, and the fluoroquinol, penicillin, beta-lactams, amoxicillin, ampicillin, azlocillin, carbenicillin, mezlocillin, nafcillin, oxacillin, piperacillin, ticarcillin, ceftazidime, ceftizoxime, ceftriaxone, cefuroxime, cephalexin, cephalothin, imipenen, aztreonam, gentamicin, netilmicin, tobramycin, tetracyclines, sulfonamides, macrolides, erythromycin, clarithromycin, azithromycin, polymyxin B, ceftiofure, cefazoline, cephapirin, and clindamycin.

14. The use of claim 10 for disinfecting a surface selected from the group consisting of a wall, a floor, a ceiling, a medical device, a medical furniture, a surgical device, a prosthesis, an orthesis, a biological fluid delivery container, (e.g. blood bag, ophtalmic drops bottle), a food processing device, a food collecting device, and a tube.

**Patentansprüche**

1. Zusammensetzung zur Hemmung von β-Lactamase in einem Mikroorganismus, umfassend Ampicillin, wobei das Ampicillin in einer Konzentration von etwa 4 μg/ml vorliegt, und ein Protein, das aus der Gruppe ausgewählt ist, die aus einem Lactoferrin, einem Lactoferricin oder einem Fragment davon besteht, in Verbindung mit einem annehmbaren Träger.

2. Zusammensetzung zur Hemmung von β-Lactamase in einem Mikroorganismus, umfassend Cefazolln, wobei das Cefazolin in einer Konzentration von etwa 0,5 μg/ml vorliegt, und ein Protein, das aus der Gruppe ausgewählt ist, die aus einem Lactoferrin, einem Lactoferricin oder einem Fragment davon besteht, In Verbindung mit einem annehmbaren Träger.

3. Zusammensetzung zur Hemmung von β-Lactamase in einem Mikroorgunlsmus, umfassend Neomycln, wobei das Neomycin in einer Konzentration von 0,125 μg/ml bis 1 μg/ml vorliegt, und eln Protein, das aus der Gruppe ausgewählt Ist, die aus einem Lactoferrin, einem Lactoferricin oder einem Fragment davon besteht, in Verbindung mit einem annehmbaren Träger.

4. Zusammensetzung zur Hemmung von β-Lactamase In einem Mikroorganismus, umfassend Novobiocin, Penicillin und ein Protein, das aus der Gruppe ausgewählt ist, die aus einem Lactoferrin, einem Lactoferricin oder einem Fragment davon besteht, in Verbindung mit einem annehmbaren Träger.

5. In-vitro-Verfahren zum Desinfizieren und/oder Verhindern der Infektion einer oberfläche durch einen β-Lactamase erzeugenden Mikroorganismus, wobei das Verfahren die Behandlung der Oberfläche mit einer wirksamen Menge Lactoferrin oder Lactoferricin umfasst.

6. Verfahren gemäß Anspruch 5, das weiterhin die Behandlung mit wenigstens einem Antibiotikum umfasst.

7. Verfahren gemäß Anspruch 5, wobei der Mikroorganismus aus der Gruppe ausgewählt ist, die aus Staphyloccccus, Streptococcus, Micrococcus, Pertococcus, Peptostreptococcus, Enterococcus, Bacillus, Clostridium, Lactobacillus, Listeria, Erysipelothrix, Propionibacterium, Eubacterium, Corynobacterium, Mycoplasma, Ureaplasma, Streptomyces, Haemophilus, Nasseria, Elkenella, Moraxellus, Actinobacillus, Pasteurella, Bacteroides,Fuscbacterium, Prevotella, Porphyromonas, Veillonella, Treponema, Mitsuokela, Capnocytophage, Campylobacter, Klebslella, Shigella, Proteus und Vibriae besteht.

8. Verfahren gemäß Anspruch 6, wobei das Antibiotikum aus der Gruppe ausgewählt Ist, die aus Aminoglycosiden, Vancomycin, Rifampin, Lincomycin, Chloramphenicol und Fluorochlnol, Penicillin, beta-Lactamen, Amoxcillin, Ampicillin, Azlocillin, Carbenicillin, Mezlocillin, Nafcillin, Oxacillin, P-paraclilin, Ticarcillin, Ceftazidim, Cefticoxim, Ceftriaxon, Cefuroxim, Cephalexin, Cephalothin, Imipenen, Aztreonam, Gentamicin, Nedimicin, Tobramycin, Tetracyclinen, Sulfonamiden, Mecrollden, Erythromycin, Clarithromycin, Azithramycin, Polymyxin B, Ceftiofur, Cefazolin, Cephapirin und Clindamycin besteht.

**9.** Verfahren gemäß Anspruch 5, wobei die Oberfläche aus der Gruppe ausgewählt ist, die aus einer Wand, einem Boden, einer Decke, einer medizinischen Vorrichtung, einem medizinischen Möbel, einer chirurgischen Vorrichtung, einer Prothese, einer Orthese, einem Behälter zur Abgabe einer biologischen Flüssigkeit (z.B. Blutbeutel, Augentropfenflasche), einer Lebensmlttel-Verarbeitungsvorrichtung, einer Lebensmittel-Sammelvorichtung und einer Sonde besteht.

**10.** Verwendung von Lactoferrin und/oder Lactoferricin bel der Herstellung eines Medikaments zur Prävention und/oder Behandlung von Infektionen, die durch einen β-Lactamase erzeugenden Mikroorganismus verursacht werden.

**11.** Verwendung gemäß Anspruch 10, wobei das Lactoferrin und/oder Lactoferricin in Kombination mit wenigstens einem Antibiotikum vorliegt.

**12.** Verwendung gemäß Anspruch 10, wobei der Mikroorganismus aus der Gruppe ausgewählt ist, die aus Staphylococcus, Streptococcus, Micrococcus, Peptococcus, Peptostreptococcus, Enterococcus, Bacillus, Clorstridium, Lactobacillus, Listeria, Erysipelothrix, Propionibacterium, Eubacterium, Corynobacterium, Mycoplasma, Ureaplasma, Streptomyces, Haemophilus, Nesseria, Eikenella, Moraxellus, Actinobacillus, Pasteurella, Bacteroides, Fusobacterium, Prevotella, Porphyromonas, Veillonella, Treponema, Mitsuokella, Capnocytophaga, Campylobacter, Klebsiella, Shigella, Proteus und Vibriae besteht.

**13.** Verwendung gemäß Anspruch 11, wobei das Antibiotikum aus der Gruppe ausgewählt ist, die aus Aminoglycosiden, Vancomycin, Rifampin, Lincom /cln, Chloramphenicol und Fluorochinol, Penicillin, beta-Lactamen, Amoxicillin, Ampicillin, Aziocillin, Carbenicillin, Mezlocillin, Nafcillin, Oxacillin, Piperacillin, Ticarcillin, Ceftazidim, Ceftizoxim, Ceftriaxon, Cefuroxirn, Cephalexin, Cephalothln, Imipenen, Aztreonam, Gentamicin, Netilmicin, Tobramycin, Tetracyclinen, Sulfonamiden, Macrollden, Erythromycin, Clarithromycin, Azithromycin, Polymyxin B, Ceftlofur, Cefazolln, Cephapirin und Clindamycin besteht.

**14.** Verwendung gemäß Anspruch 10 zum Desinfizieren einer Oberfläche, die aus der Gruppe ausgewählt ist, die aus einer Wand, einem Boden, einer Decke, einer medizinischen Vorrichtung, einem medizinischen Möbel, einer chirurgischen Vorrichtung, einer Prothese, einer Orthese, einem Behälten zur Abgabe einer biologischen Flüsslgkelt (z.B. Blutbeutel, Augentropferflasche), einer Lebensmittel-Verarbeitungsvorrichtung, einer Lebensmitte-Sammelvorrichtung und einer Sonde besteht.

## Revendications

**1.** Une composition permettant d'inhiber la β-lactamase dans un microorganisme comprenant de l'ampicilline, ladite ampicilline est à une concentration d'environ 4 μg/ml, et une protéine choisie dans le groupe constitué par une lactoferrine, une lactoferricine ou un fragment de ces dernières, en association avec un véhicule acceptable.

**2.** Une composition permettant d'inhiber la β-lactamase dans un microorganisme comprenant la céfazoline, dans laquelle ladite céfazoline est à une concentration d'environ 0,5 μm/ml, et une protéine choisie dans le groupe constitué par une lactoferrine, une lactoferricine ou un fragment de ces dernières, en association avec un véhicule acceptable.

**3.** Une composition permettant d'inhiber la β-lactamase dans un microorganisme comprenant la néomycine, dans laquelle ladite néomycine est à une concentration comprise entre 0,125 μm/ml et 1 μm/ml, et une protéine choisie dans le groupe constitué par une lactoferrine, une lactofenicine ou un fragment, de ces dernières, en association avec un véhicule acceptable.

**4.** Une composition permettant d'inhiber la β-lactamase dans un microorganisme comprenant la novobiocinc, la pénicilline et une protéine choisie dans le groupe constitué par une lactoferrine, une lactoferricine ou un fragment de ces dernières, en association avec un véhicule acceptable.

**5.** Une méthode *in vitro* permettant de désinfecter et/ou de prévenir l'infection d'une surface par un microorganisme produisant de la β-lactamase, ladite méthode comprenant le traitement de ladite surface avec une quantité efficace de lactoferrine ou de lactoferricine.

**6.** La méthode de la revendication 5 comprenant en outre le traitement avec au moins un antibiotique.

**7.** La méthode de la revendication 5, dans laquelle ledit microorganisme est choisi dans le groupe constitué par Staphylococcus, Streptococcus, Micrococcus, Peptococcus, Peptostreptococcus, Enterococcus, Bacillus, Clostridium, Lactobacillus, Listeria, Erysipelothrix, Propionibacterium, Eubacterium, Corynobacterium, Mycoplasma, Ureaplasma, Streptomyces, Heamophilus, Neisseria, Eikenella, Moraxellus, Elctinobacillus, Pasteurella, Bacteroides, Fusomicroorganisme, Prevotella, Porphyromonas, Veillonella, Treponema, Mitsuokella, Capnocytophaga, Campylobacter, Klebsiella, Shigella, Proteus et Vibrise.

**8.** La méthode de la revendication 6, dans laquelle ledit antibiotique est choisi dans le groupe constitué par les aminoglycosides, la vancomycine, la rifampine, la lincomycine, le chloramphénicol, et le fluoroquinol, la pénicilline, les bêta-lactames, l'amoxicilline, l'ampicilline, l'azlocilline, la carbénicilline, la mezlocilline, la nafcilline, l'oxacilline, la pipéracilline, la ticareilline, la ceftazidime, la ceftizoxime, la ceftriaxone, la céfuroxime, la céphalexine, la caphalothine, l'imipcnène, l'aztréoname, la gcntamicine, la nétilmicinc, la tobrarnycine, les tétracyclines, les sulfonamidcs, les macrotides, l'érythrornycine, la clarithromycine, l'azithromycine, la polymyxine B, la ceftiofure, la céfazoline, la céphapirine, et la clindamycine.

**9.** La méthode de la revendication 5, dans laquelle ladite surface est choisie parmi le groupe constitué par un mur, un plancher, un plafond, un dispositif médical, un mobilier médical, un dispositif chirurgical, une prothèse, une orthèse, un récipient pour la livraison d'un fluide biologique, (par exemple un sac pour contenir du sang, une bouteille pour gouttes ophtalmiques), un dispositif de traitement de nourriture, un dispositif pour recueillir de la nourriture, et un tube.

**10.** Usage de la lactoferrine et/ou de la lactoferricinc pour la fabrication d'un médicament destiné à la prévention et/ou au traitement d'infections causées par un microorganisme produisant de la β-lactamase.

**11.** L'usage de la revendication 10, dans laquelle ladite lactoferrine et/ou lactoferricine est en combinaison avec au moins un antibiotique.

**12.** L'usage de la revendication 10, dans lequel ledit microorganisme est choisi dans le groupe constitué par Staphylococcus, Streptococcus, Micrococcus, Peptococcus, Peptostreptococcus, Enterococcus, Bacillus, Clostridium, Lactobacillus, Listeria, Erysipelothrix, Propionibacterium, Eubacterium, Corynobacteriurn, Mycoplasma, Ureaplasma, Streptomyces, Heamophilus, Neisseria, Eikenella, Moraxellus, Actinobacillus, Pasteurella, Bacteroidcs, Fusomicroorganisme, Prcvotella, Porphyromonas, Veillonella, Treponema, Mitsuokella, Capnocytophaga, Campyfebacter, Klcbsiclla, Shigella, Proteus et Vibrise.

**13.** L'usage de la revendication 11, dans lequel ledit antibiotique est choisi dans le groupe constitué par les aminoglycosides, la vancomycine, la rifampine, la lincomycine, le chloramphénicol, et le fluoroquinol, la pénicilline, les bêtalactames, l'amoxicilline, l'ampicilline, l'azlocilline, la carbénicilline, la mezlocilline, la nafcilline, l'oxacilline, la pipéracilline, la ticarcilline, la ceftazidime, la ceftizoxime, la ceftriaxone, la céfuroxime, la céphalexinc, la caphalothine, l'imipenène, l'azstréoname, la gentamicine, la nétilmicine, la tobramycine, les tétracyclines, les sulfanamides, les macrolides, l'érythromycine, la clarithromycine, l'azithromycine, la polymyxine B, la ceftiofure, la cëfazoline, la céphapirine, et la clindamycine.

**14.** L'usage de la revendication 10 pour désinfecter une surface choisie parmi le groupe constitué par un mur, un plancher, un plafond, un dispositif médical, un mobilier médical, un dispositif chirurgical, une prothèse, une orthèse, un récipient pour la livraison d'un fluide biologique, (par exemple un sac pour contenir du sang, une bouteille pour gouttes ophtalmiques), un dispositif de traitement de nourriture, un dispositif pour recueillir de la nourriture, et un tube.

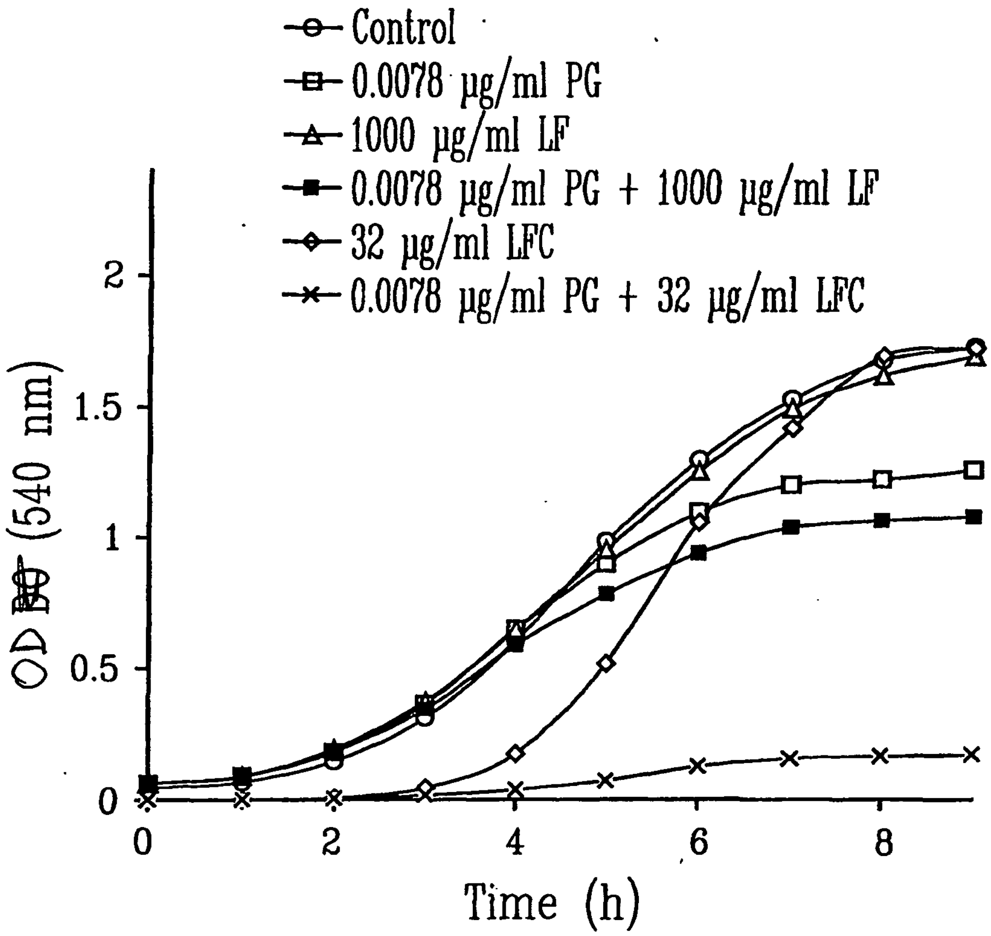
Control
0.0078 µg/ml PG
1000 µg/ml LF
0.0078 µg/ml PG + 1000 µg/ml LF
32 µg/ml LFC
0.0078 µg/ml PG + 32 µg/ml LFC
OD (540 nm)
0
0.5
1
1.5
2
0
2
4
6
8
Time (h)

FIG. 1

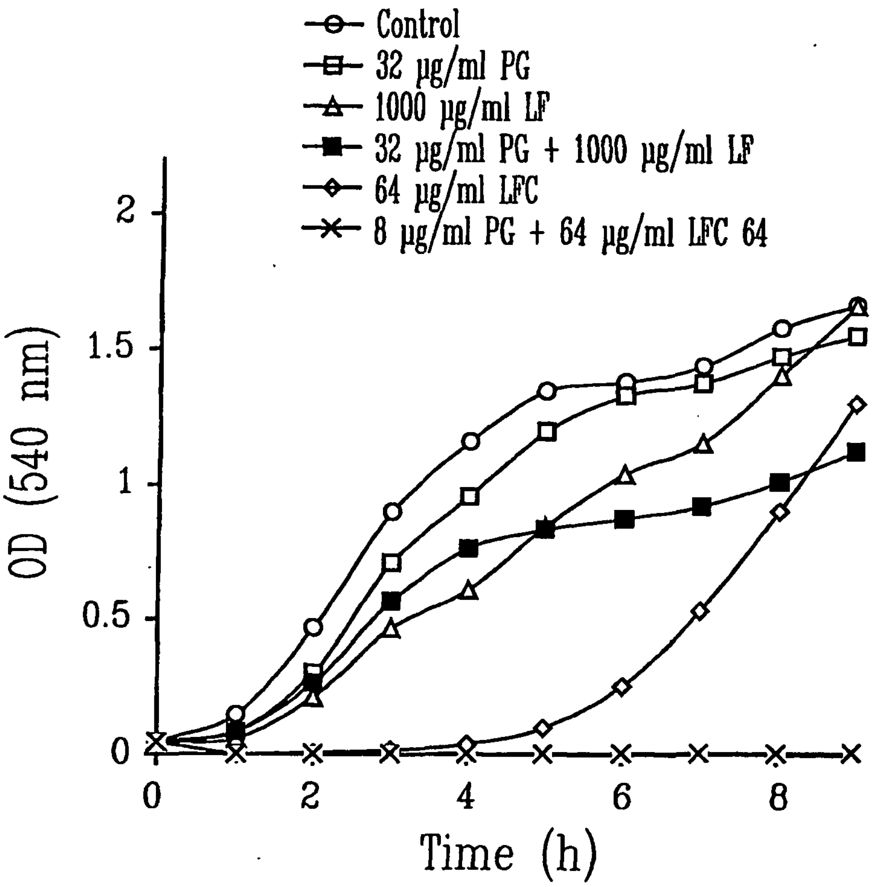
Control
32 µg/ml PG
1000 µg/ml LF
32 µg/ml PG + 1000 µg/ml LF
64 µg/ml LFC
8 µg/ml PG + 64 µg/ml LFC 64
OD (540 nm)
2
1.5
1
0.5
0
0
2
4
6
8
Time (h)

FIG. 2

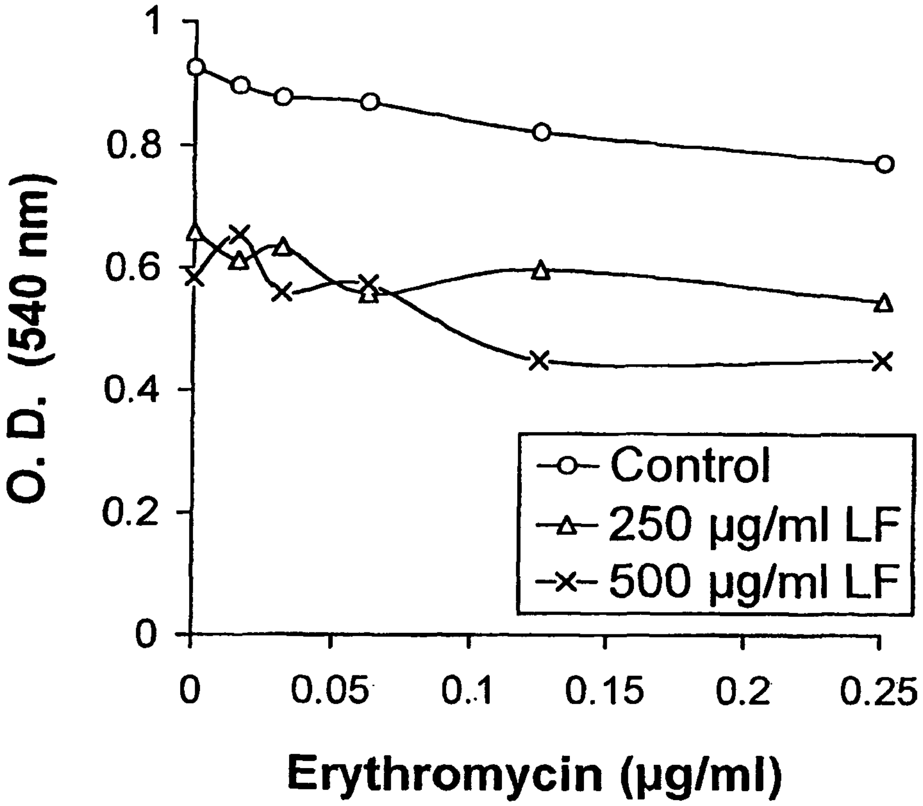
1
0.8
0.6
0.4
0.2
0
O. D. (540 nm)
Control
250 µg/ml LF
500 µg/ml LF
0
0.05
0.1
0.15
0.2
0.25
Erythromycin (µg/ml)

FIG. 3

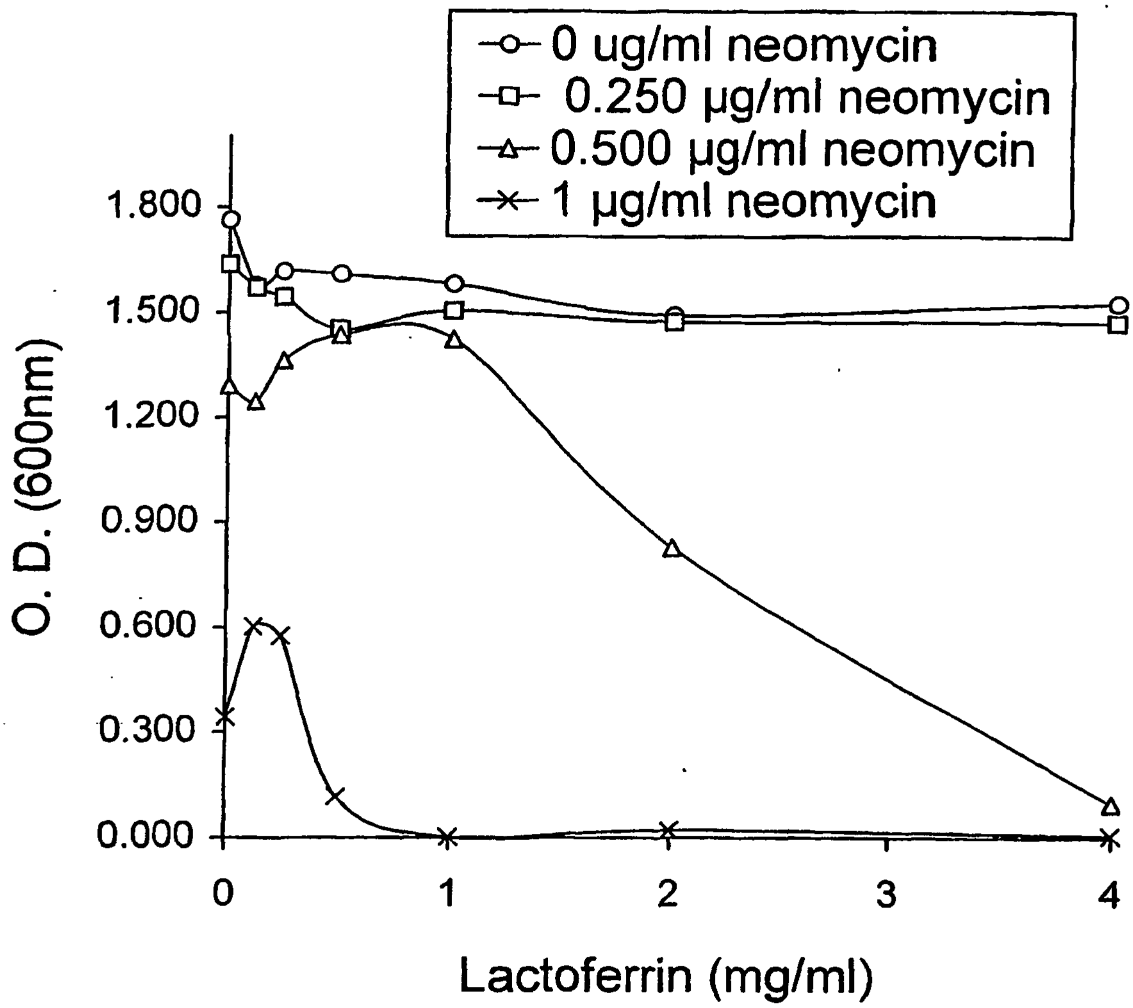
0 ug/ml neomycin
0.250 µg/ml neomycin
0.500 µg/ml neomycin
1 µg/ml neomycin
O. D. (600nm)
1.800
1.500
1.200
0.900
0.600
0.300
0.000
0
1
2
3
4
Lactoferrin (mg/ml)

FIG. 4

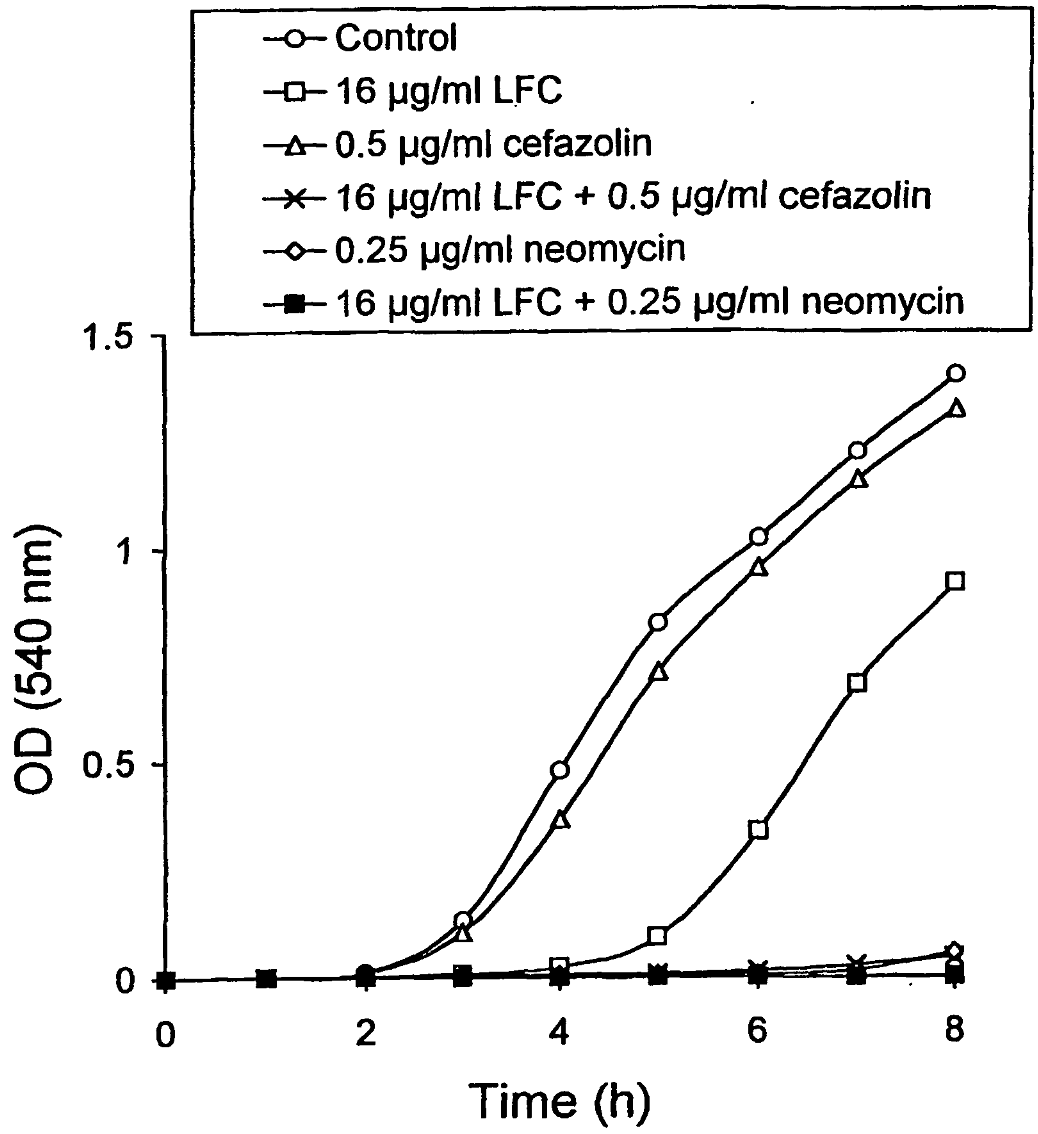
Control
16 µg/ml LFC
0.5 µg/ml cefazolin
16 µg/ml LFC + 0.5 µg/ml cefazolin
0.25 µg/ml neomycin
16 µg/ml LFC + 0.25 µg/ml neomycin
1.5
1
0.5
0
OD (540 nm)
0
2
4
6
8
Time (h)

FIG. 5

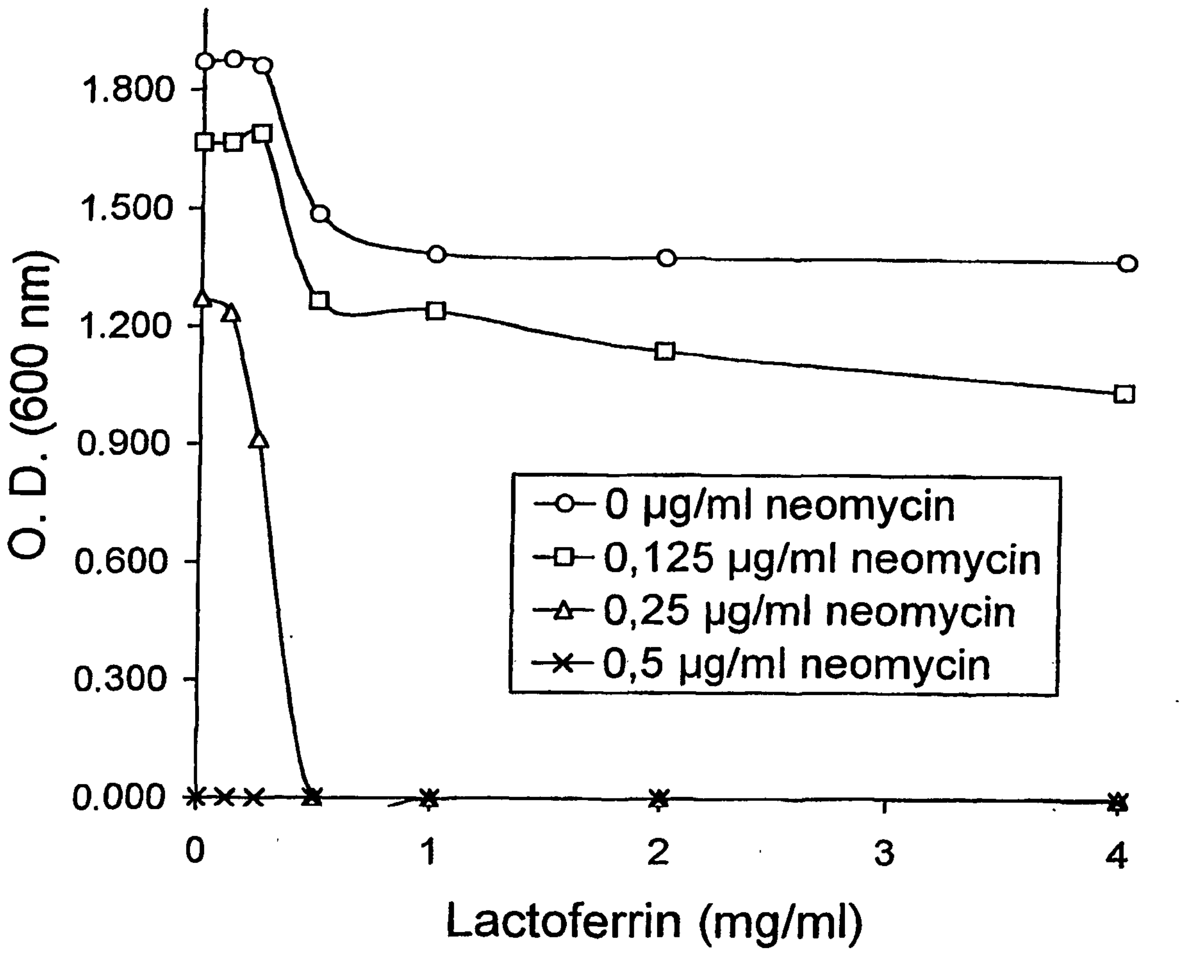
O. D. (600 nm)
1.800
1.500
1.200
0.900
0.600
0.300
0.000
0
1
2
3
4
Lactoferrin (mg/ml)
0 µg/ml neomycin
0,125 µg/ml neomycin
0,25 µg/ml neomycin
0,5 µg/ml neomycin

FIG. 6

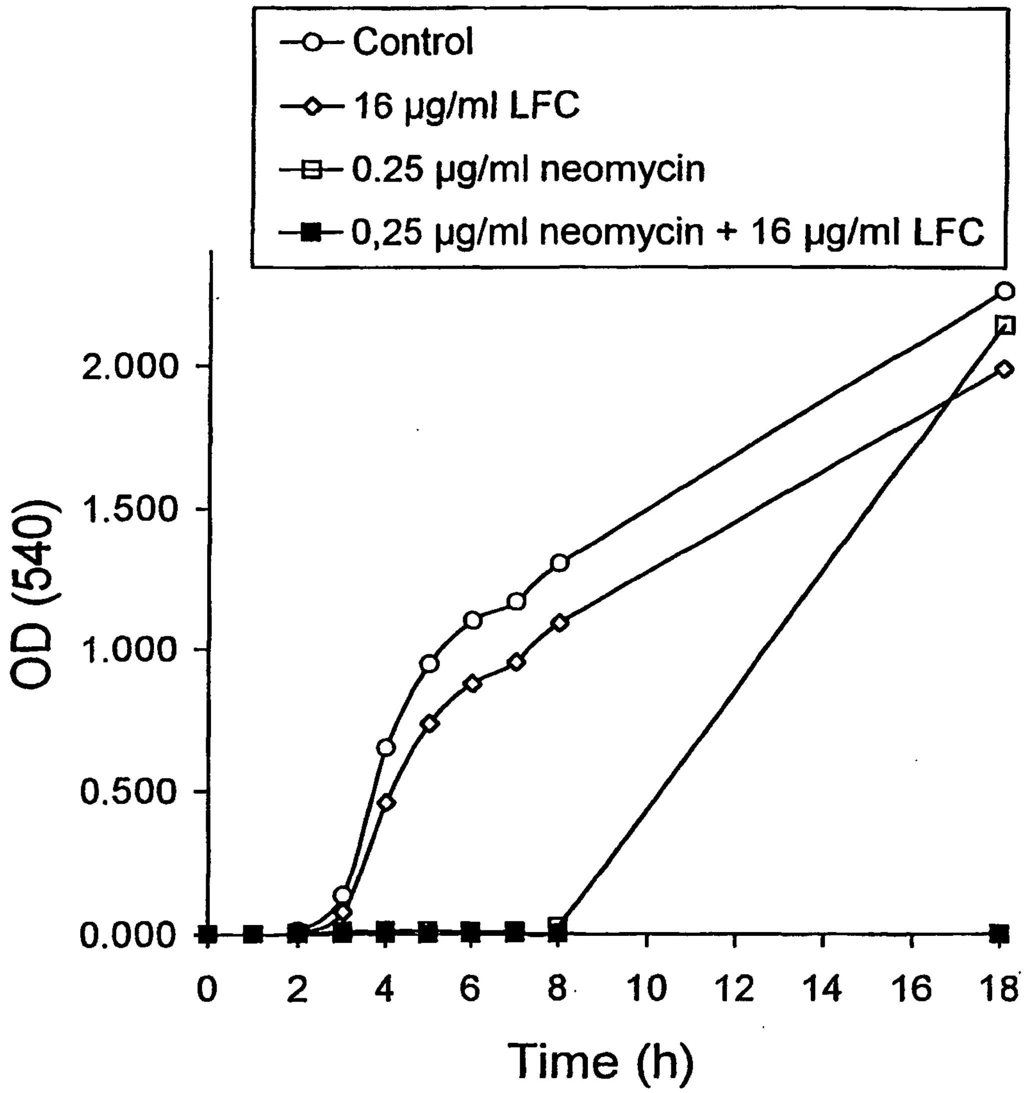
Control
16 µg/ml LFC
0.25 µg/ml neomycin
0,25 µg/ml neomycin + 16 µg/ml LFC
OD (540)
2.000
1.500
1.000
0.500
0.000
0
2
4
6
8
10
12
14
16
18
Time (h)

FIG. 7

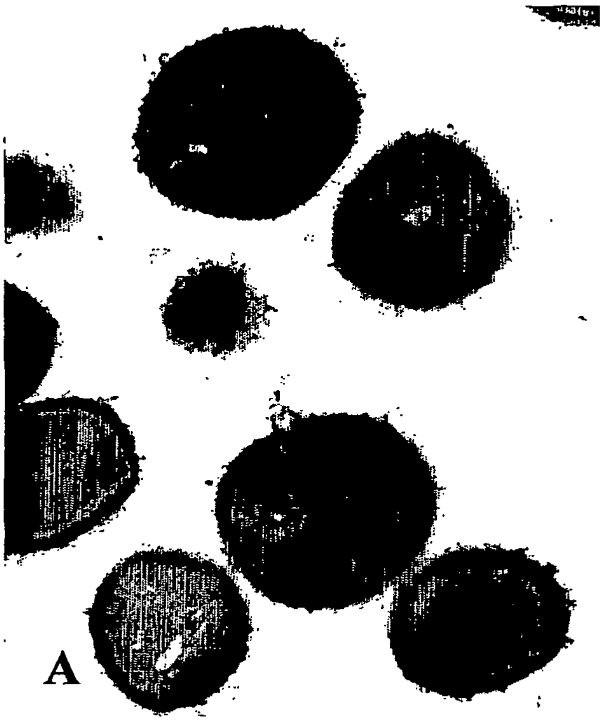

FIG. 8A

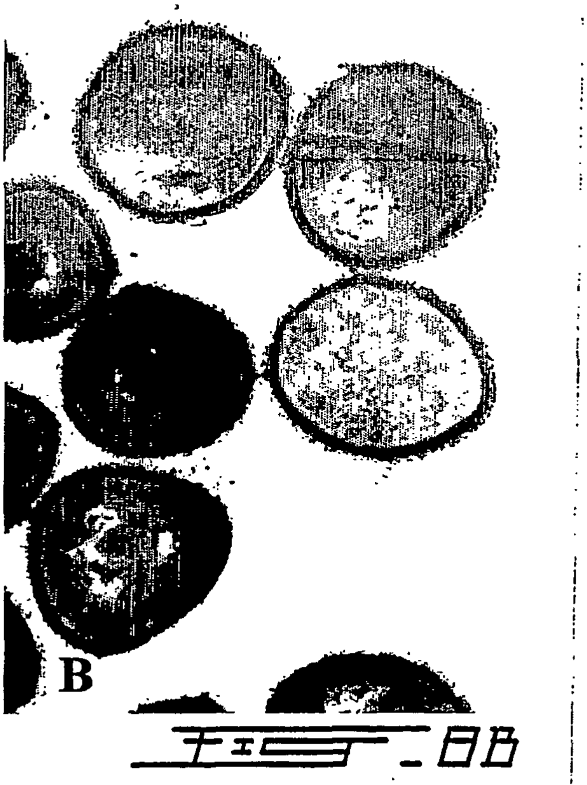

FIG. 8B

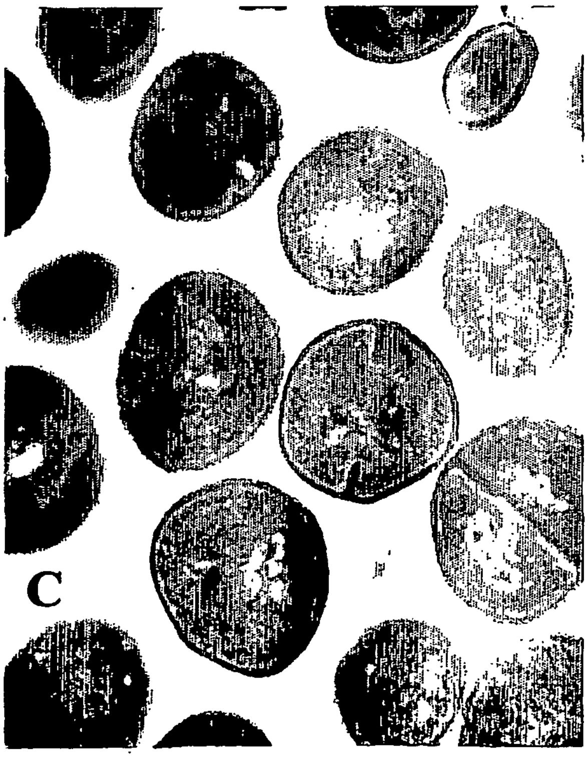

FIG. 8C

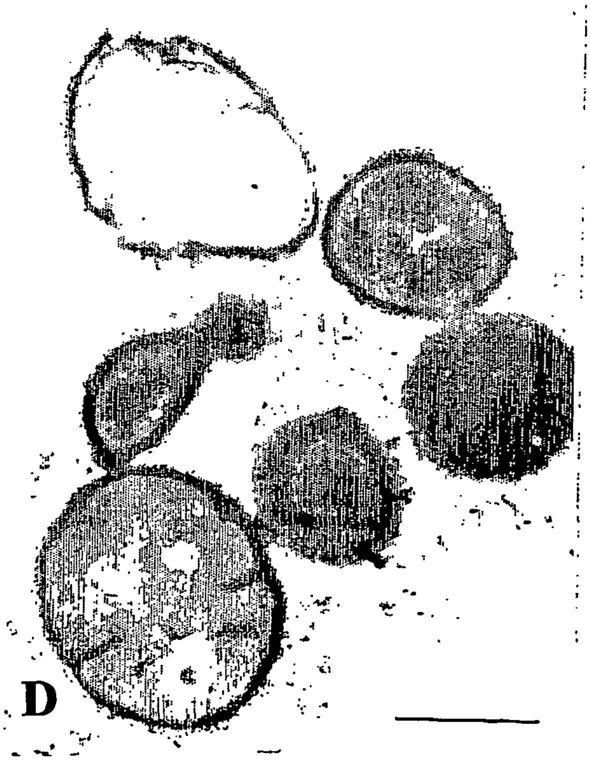

FIG. 8D

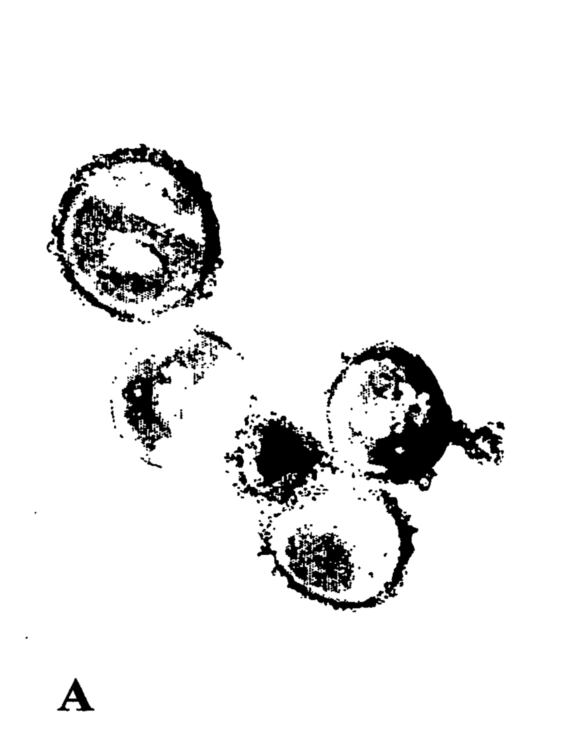


FIG. 9A

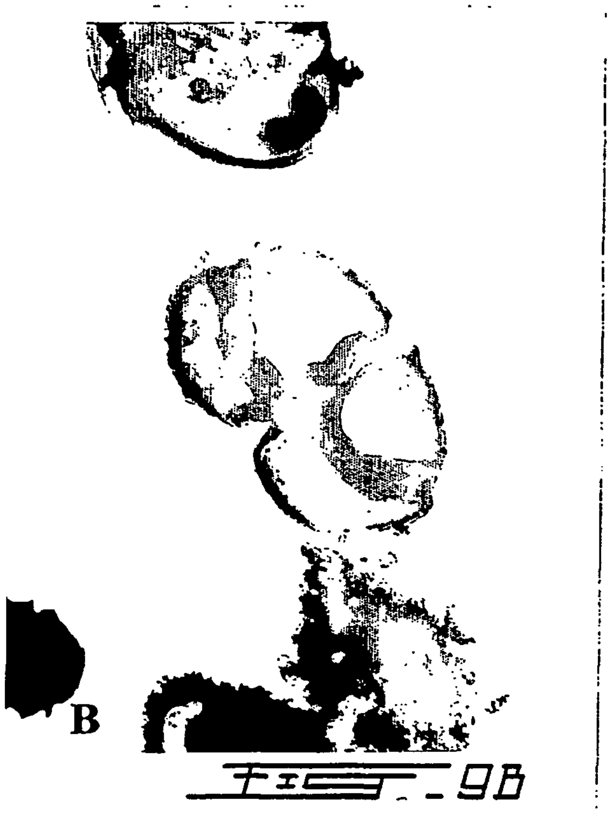


FIG. 9B

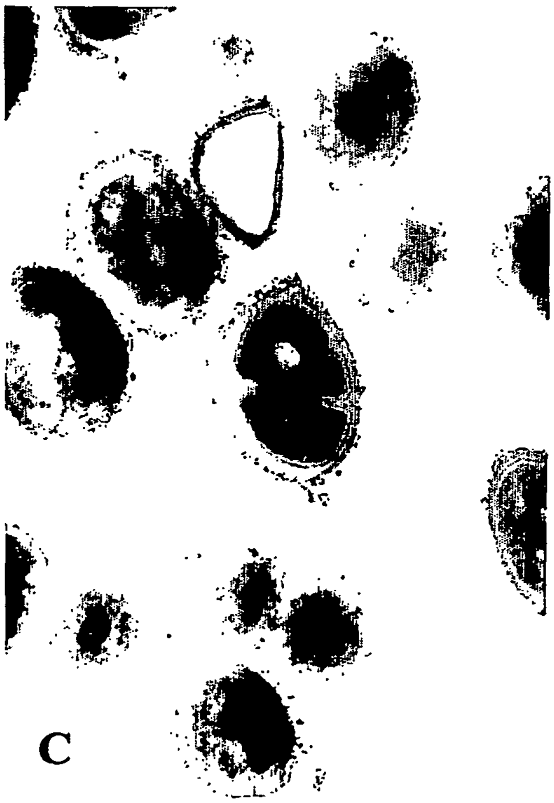


FIG. 9C

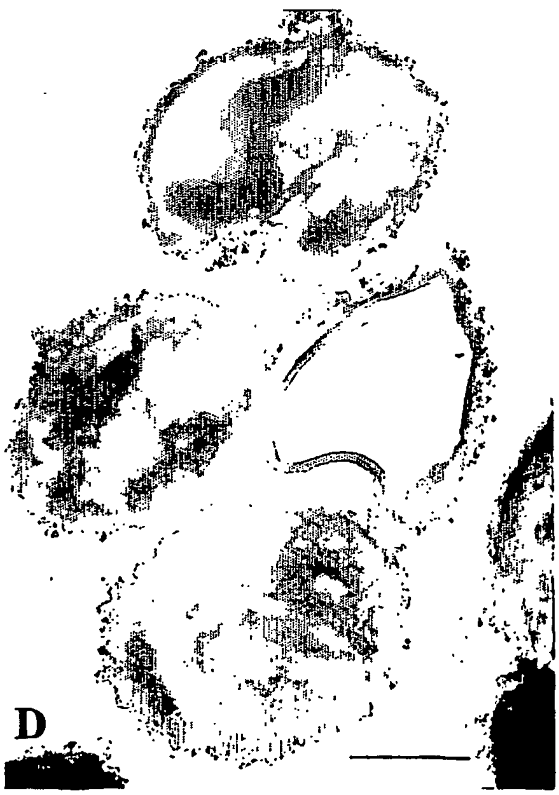


FIG. 9D

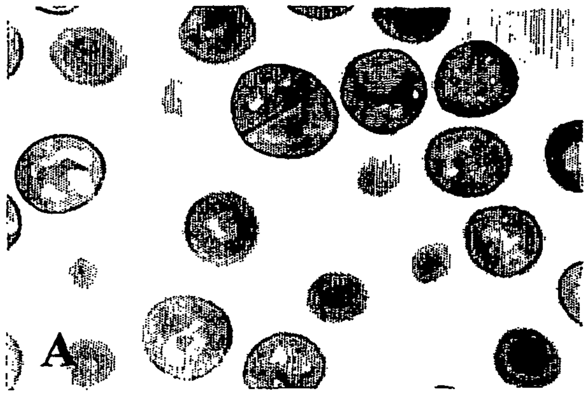


FIG. 10A

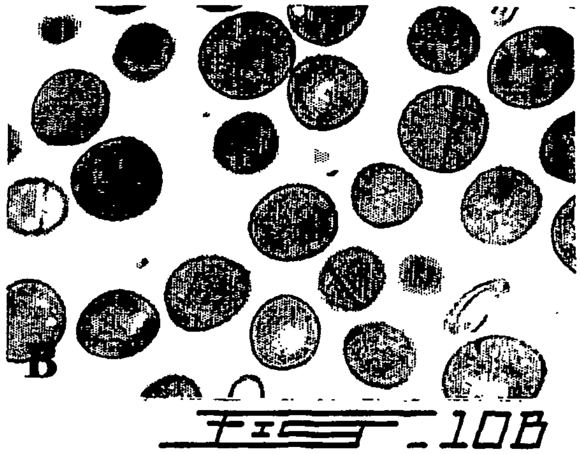


FIG. 10B

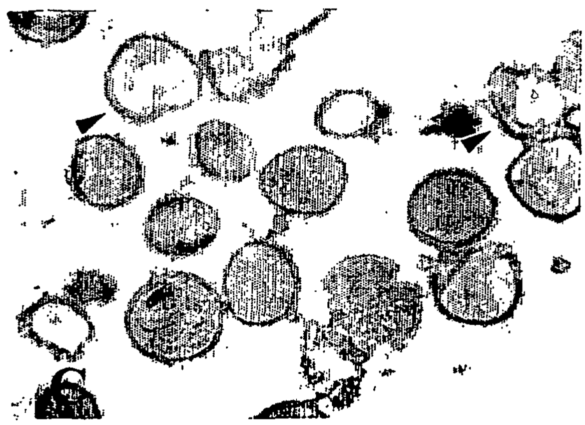


FIG. 10C

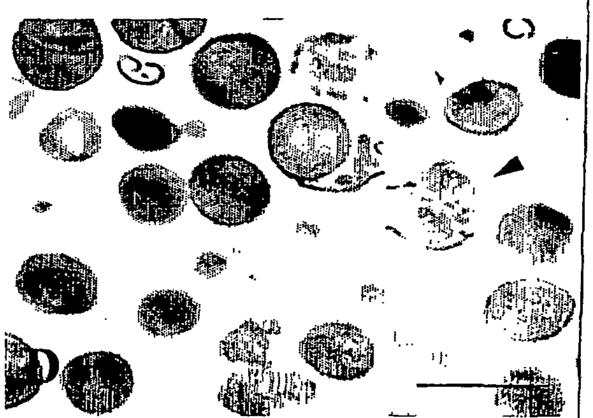


FIG. 10D

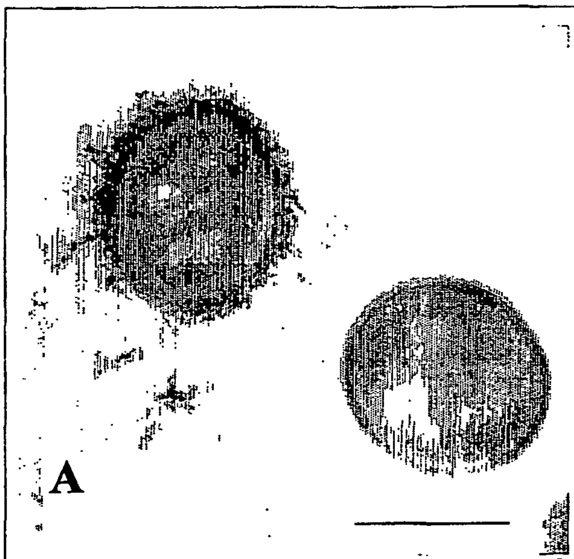


FIG. 11A

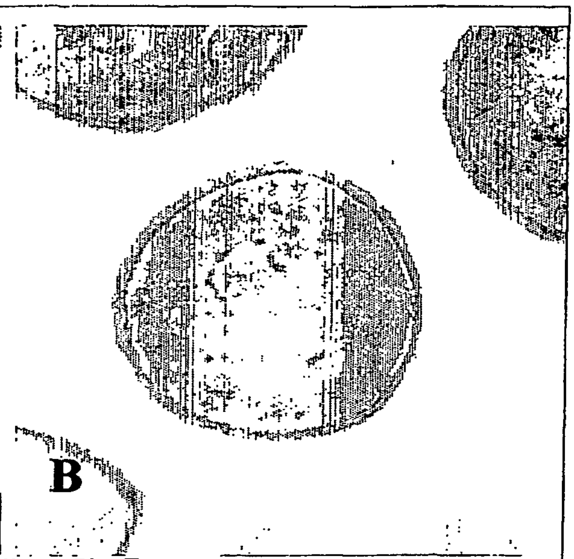


FIG. 11B

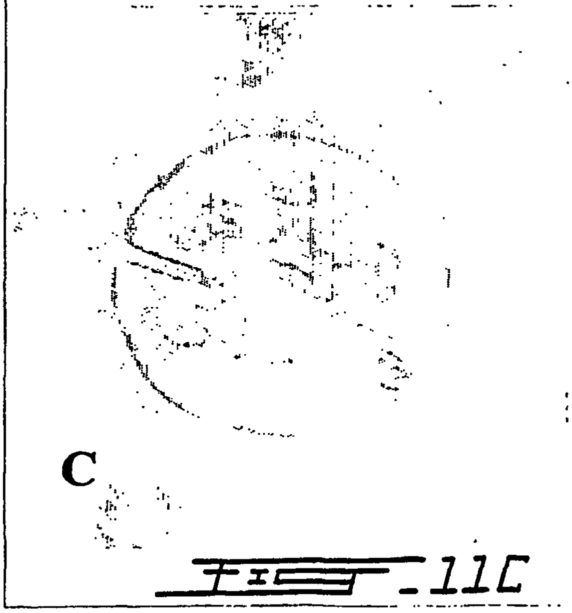


FIG. 11C

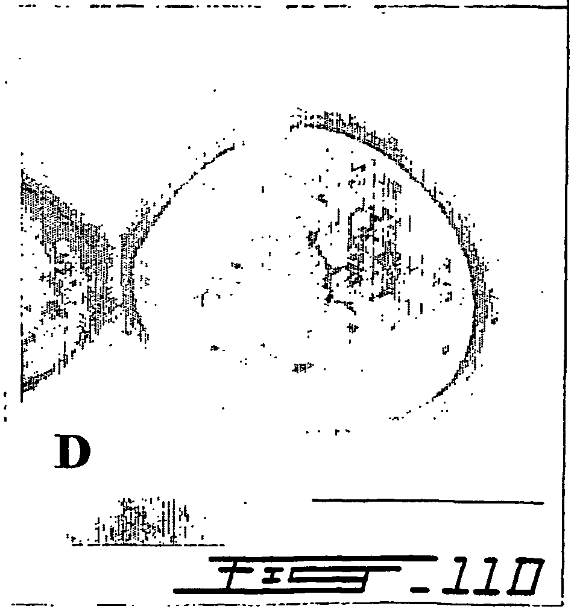


FIG. 11D

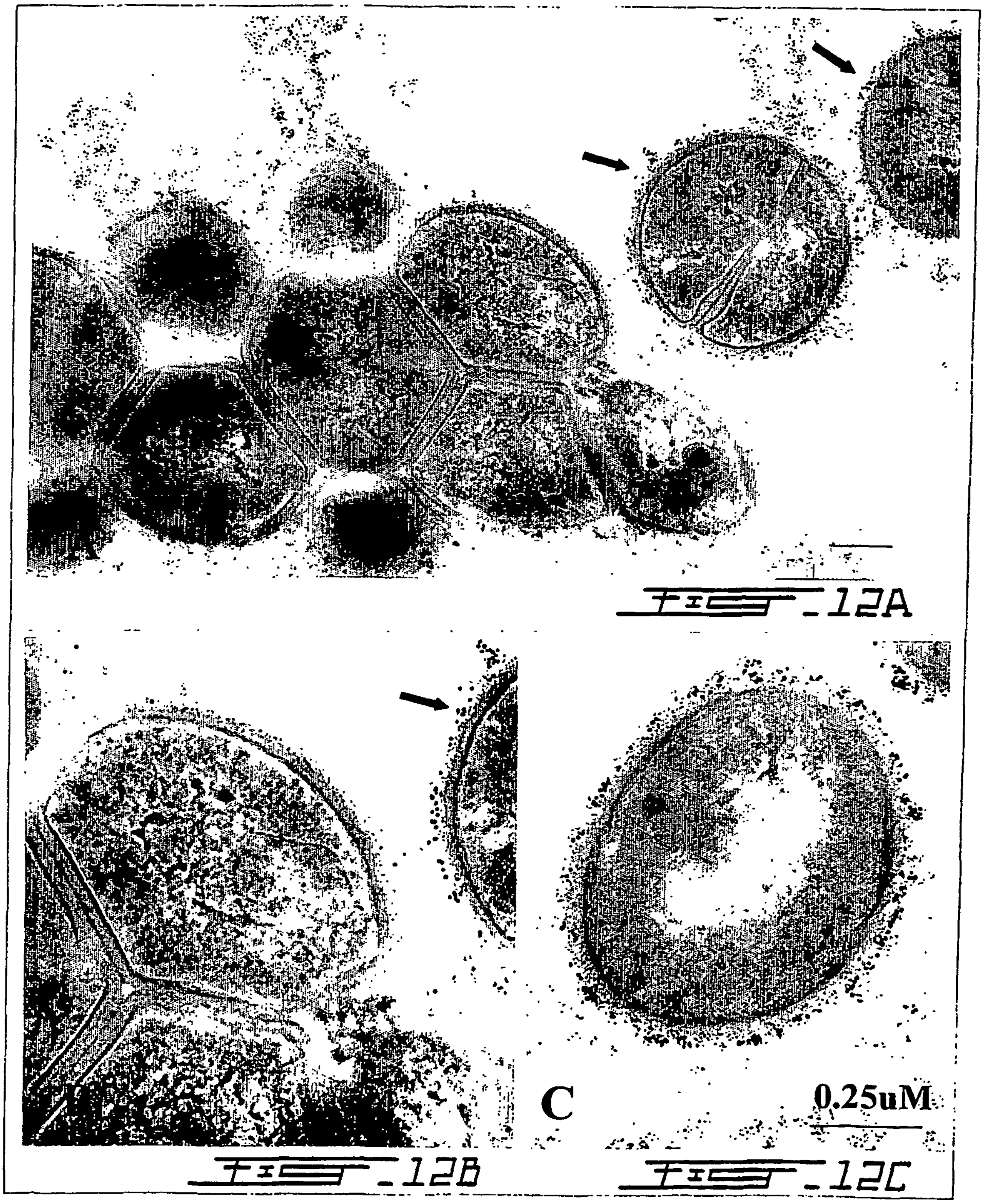


FIG. 12A

FIG. 12B

FIG. 12C

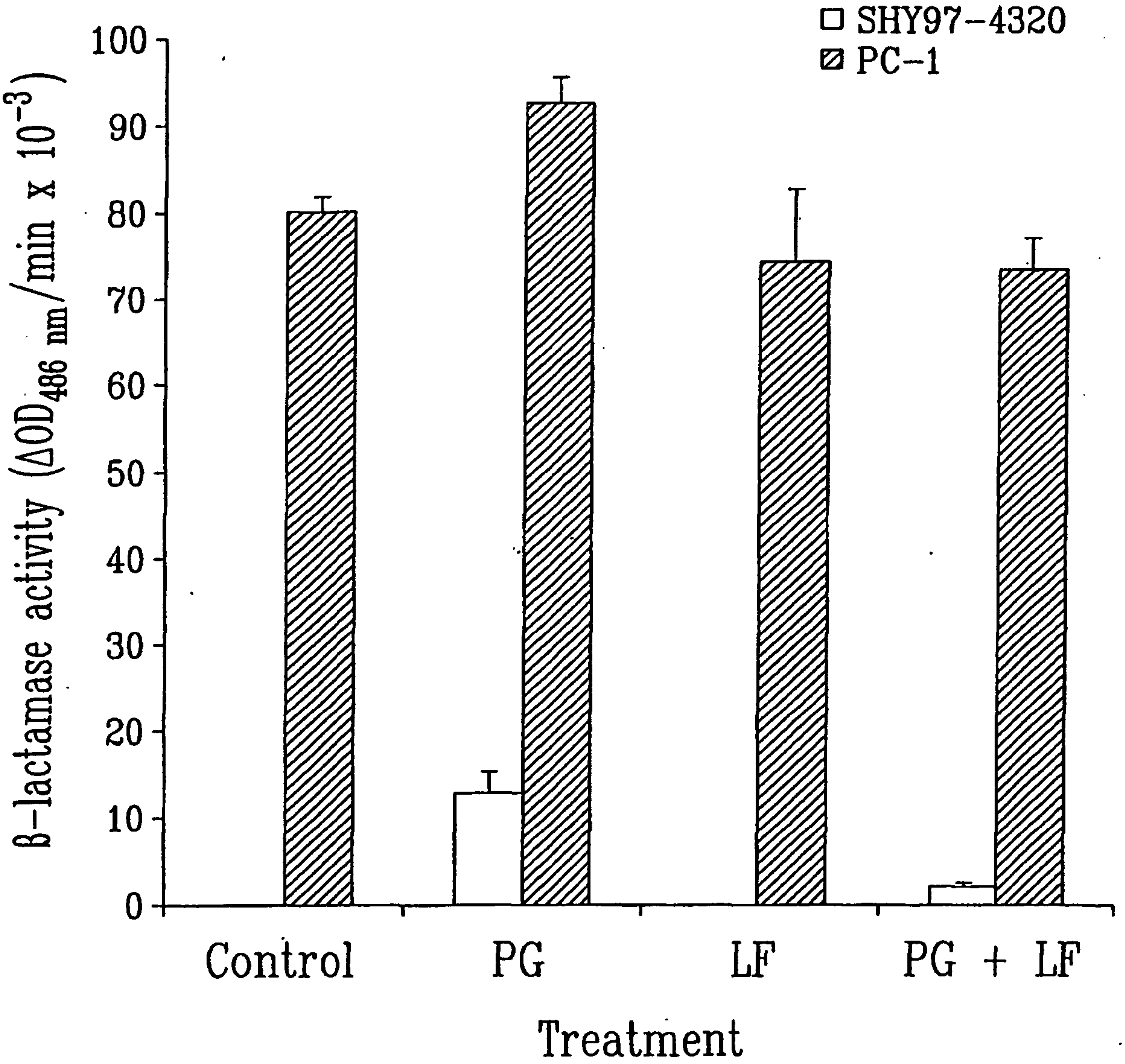
SHY97-4320
PC-1
ß-lactamase activity (ΔOD486 nm/min x 10-3)
100
90
80
70
60
50
40
30
20
10
0
Control
PG
LF
PG + LF
Treatment

FIG. 13

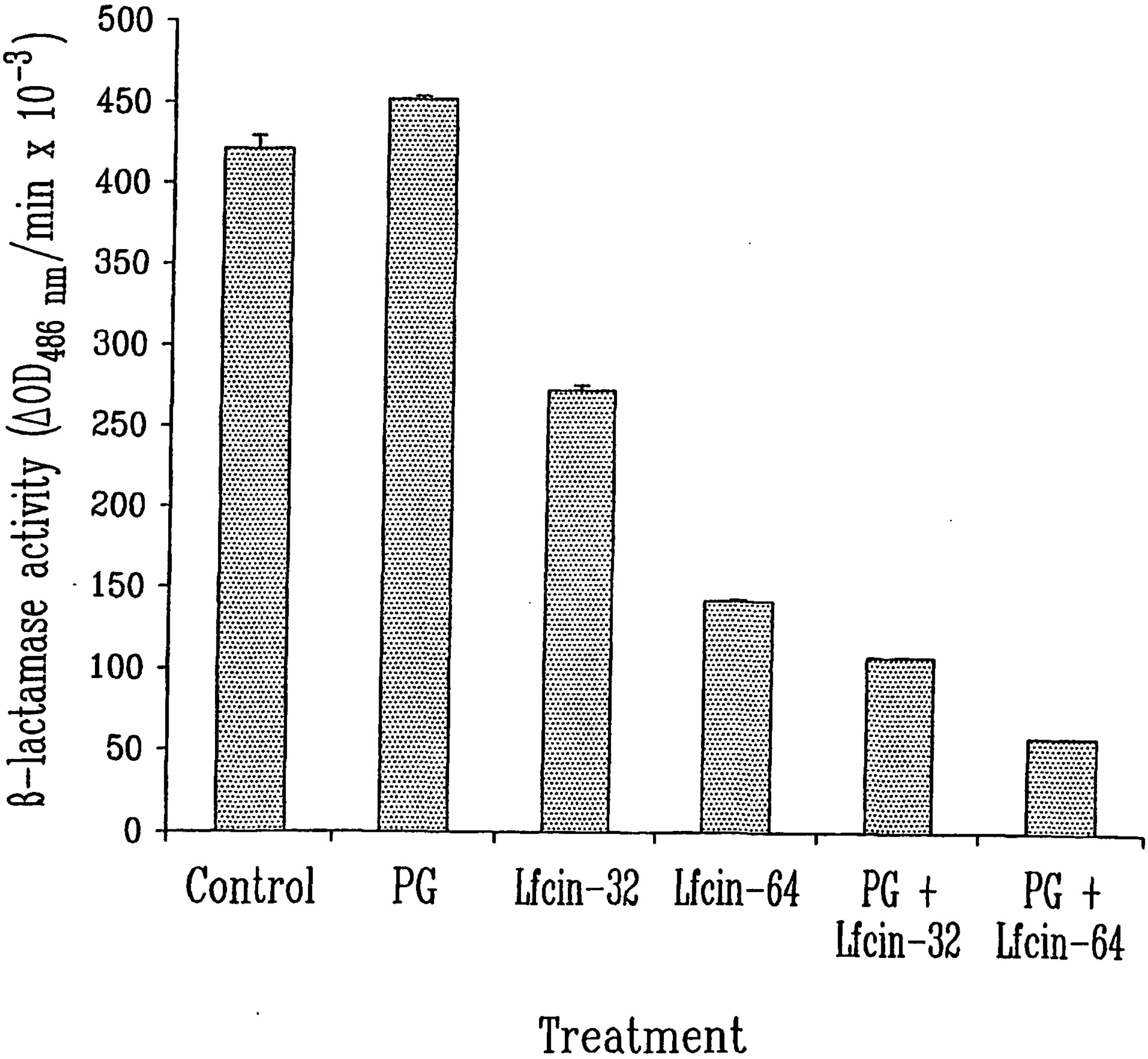
β-lactamase activity (ΔOD486 nm/min x 10-3)
500
450
400
350
300
250
200
150
100
50
0
Control
PG
Lfcin-32
Lfcin-64
PG + Lfcin-32
PG + Lfcin-64
Treatment

FIG. 14

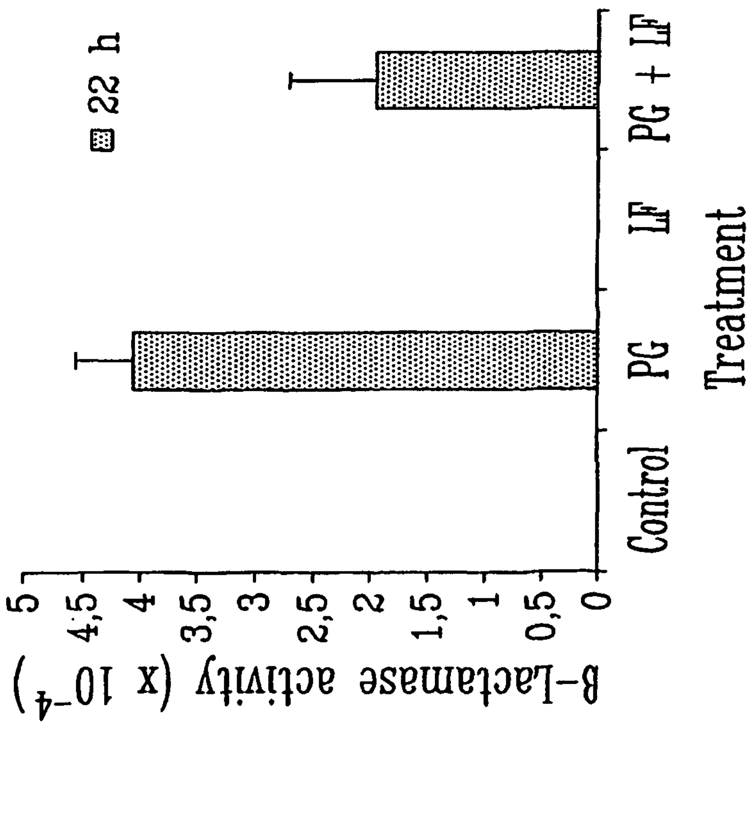


FIG. 15A

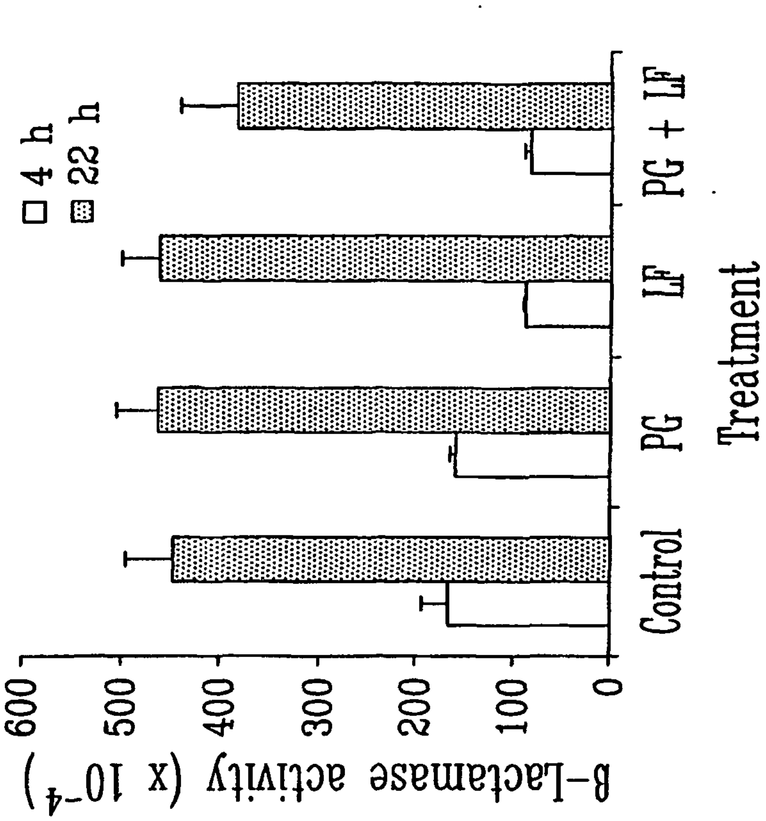


FIG. 15B

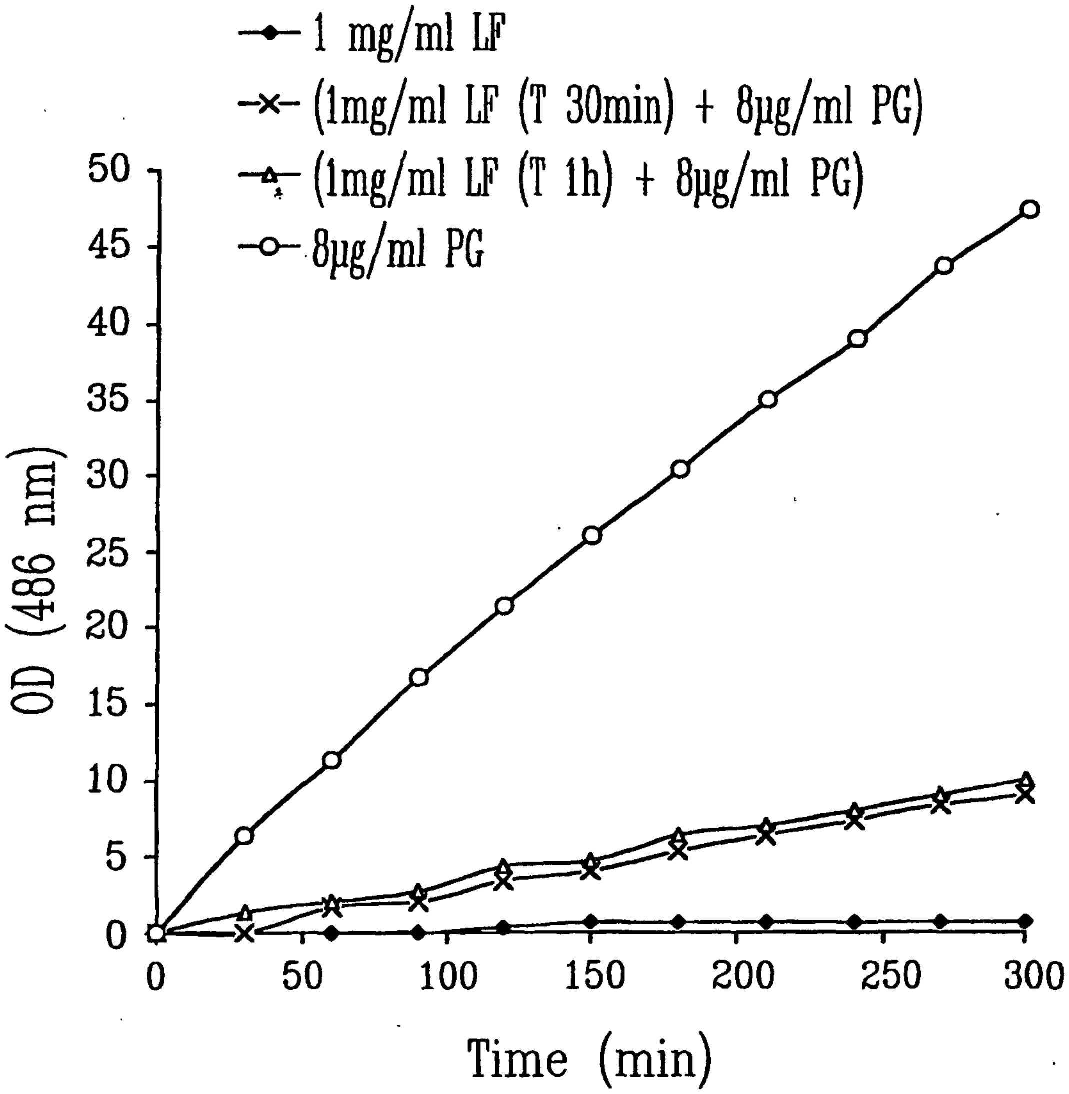
1 mg/ml LF
(1mg/ml LF (T 30min) + 8µg/ml PG)
(1mg/ml LF (T 1h) + 8µg/ml PG)
8µg/ml PG
OD (486 nm)
0
5
10
15
20
25
30
35
40
45
50
0
50
100
150
200
250
300
Time (min)

FIG. 16

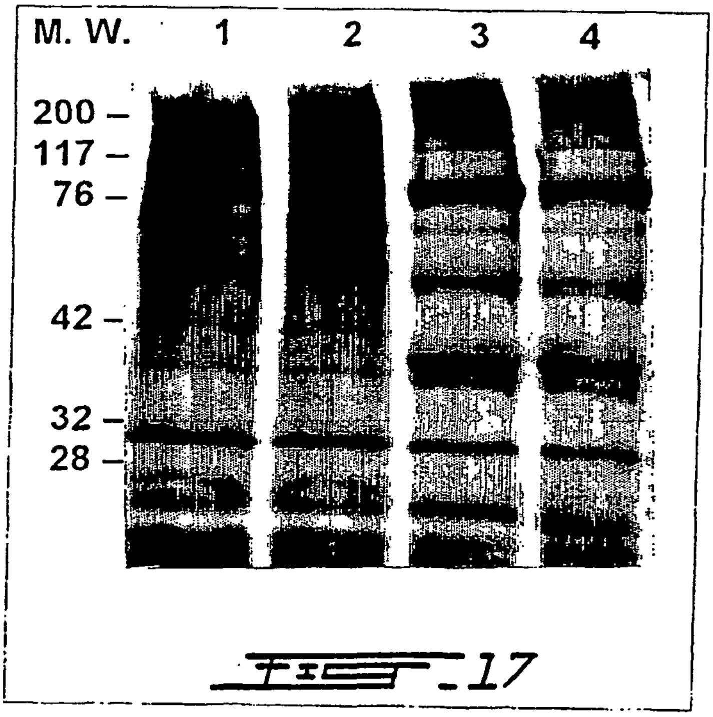


FIG. 17

SEQUENCE LISTING

<110> Sa Majesté La Reine du Chef du Canada Agriculture et Agroalimentaire Canada DIARRA, Moussa S. LACASSE, Pierre PETITCLERC, Denis

<120> METHOD AND COMPOSITION FOR TREATMENT AND/OR PREVENTION OF ANTIBIOTIC-RESISTANT MICROORGANISM INFECTIONS

<130> 14654-lpct

<150> US 60/172,577
<151> 1999-12-20

<160> 5

<170> FastSEQ for Windows Version 3.0

<210> 1
<211> 20
<212> DNA
<213> Artificial Sequence

<220>
<223> Reverse primer for BlaZ gene of S. Aureus

<400> 1

tagtcttttg gaacaccgtc 20

<210> 2
<211> 20
<212> DNA
<213> Artificial Sequence

<220>
<223> Forward primer for BlaZ gene of S. Aureus

<400> 2

acagttcaca tgccaaagag 20

<210> 3
<211> 27
<212> DNA
<213> Artificial Sequence

<220>
<223> Forward primer for quantifying beta-lactamase BlaZ RNA

<400> 3

aattaaatta ctattcgcca aagagca 27

<210> 4
<211> 24
<212> DNA
<213> Artificial Sequence

<220>
<223> Reverse primer for quantifying beta-lactamase BlaZ RNA

<400> 4

```
tgcttaattt tccatttgcg ataa                                                24
```

<210> 5
<211> 22
<212> DNA
<213> Artificial Sequence

<220>
<223> TAQMan probe

<400> 5

```
acgcctgctg ctttcggcaa ga                                                  22
```